# EUROPEAN PATENT APPLICATION

(11) **EP 4 339 202 A1**
(43) Date of publication of application: **20.03.2024**
(21) Application number: 22196120.4
(22) Date of filing: 16.09.2022
(51) Int. Cl.: C07K 4/00, C07K 14/00

(54) **FUSION PROTEINS COMPRISING GG REPEAT SEQUENCES II**

(71) Applicant: NUMAFERM GmbH, 40225 Düsseldorf (DE)
(72) Inventor: Schwarz, Christian, 41468 Neuss (DE); Stock, Janpeter, 40225 Düsseldorf (DE); Montoya, José, 40225 Düsseldorf (DE)
(74) Representative: Maiwald GmbH

(57) **Abstract**

The present invention relates to polypeptides comprising a first amino acid sequence comprising one or more GG repeat sequences and a peptide or polypeptide of interest in form of a fusion protein that exhibits increased renaturation efficiency and optionally also improved expression. Also encompassed are nucleic acids encoding these polypeptides, host cells that comprise said nucleic acids, and methods for protein expression and renaturation using said nucleic acids, host cells and polypeptides.

## Description

### FIELD OF THE INVENTION

The present invention lies in the field of molecular biology, recombinant peptide and protein expression and relates to amino acid sequences that support the refolding and renaturation of a peptide or protein of interest recombinantly expressed as a fusion protein.

### BACKGROUND OF THE INVENTION

To date, recombinant protein/enzyme production for use in industrial processes is widely established. It is expected that in the future more and more industrial processes that are currently based on traditional chemistry will be adapted to involve recombinant technologies.

The type 1 secretion system (T1SS) is a relatively simple yet highly conserved secretion strategy used throughout Gram-negative bacteria to translocate small substrates as well as extremely large proteins to the extracellular environment. Much of our current knowledge of T1SS has been gathered over the last several decades from studies of the T1SS toxins HlyA and CyaA of *Escherichia coli* and *Bordetella pertussis,* respectively. These toxins, and the majority of type 1 secreted proteins, belong to the repeats-in-toxin (RTX) family (Welch (1991), Mol Microbiol 5:512-528).

While the technologies based on the T1SS, in particular fusion proteins of peptides and proteins to be expressed with HlyA are known in the art, for example from the international patent publications WO 2013/057312 A1 and WO 2014/170430 A1, and are commercially available, it has been found that in various instances high yields are prevented by suboptimal solubility or susceptibility to proteolysis of the fusion constructs. Thus, there still exists need for further optimized methods that allow more efficient production of peptides and proteins that overcome some of the drawbacks of existing methods.

### SUMMARY OF THE INVENTION

The present invention is based on the inventor's surprising finding that two or more so-called GG repeats, short amino acid sequences of the general consensus sequence GGxGxDxUx derived from a specific class of naturally occurring proteins can be artificially combined and optionally inserted into a scaffold to form a novel, non-natural amino acid sequence (herein referred to as first amino acid sequence) that, if fused to another peptide or polypeptide of interest that is to be recombinantly produced by expression of a fusion protein in a host cell, provides for increased renaturation efficiency, while at the same time providing for good stability and/or solubility. Interestingly, it was found that efficiency of renaturation of the peptide or polypeptide of interest by the GG-repeat sequence containing part is dominated by the specific GG repeat sequence in the second position (based on N- to C-terminal orientation) and correlates to the relative frequency of said GG repeat in nature, whereas the exact sequence of the first GG repeat (in N-to C-terminal orientation) has only little influence on renaturation efficiency. Based on this finding, artificial GG repeat combinations could be developed that performed significantly better than naturally occurring GG repeat combinations. Interestingly, it was found that by choosing such an approach a sequence element of only two GG repeat sequences could replace a natural sequence of four GG repeats and GG repeat-like sequences with improved or at least the same renaturation efficiency. It needs to be noted that the possibility to shorten the renaturation tag while maintaining renaturation efficiency provides for an improvement, as the relative yields of the peptide/polypeptide of interest will still be higher.

The newly developed amino acid sequences allow for the production of the fusion protein in form of high titer inclusion bodies with high initial purities after extraction while at the same time protecting fused peptides/proteins from degradation, e.g. proteolysis, desamidation, truncation, oxidation and the like and, most importantly, allowing their renaturation from a denatured into a properly folded functional state (by means of using the novel sequences as a fusion tag).

In a first aspect, the present invention is therefore directed to an isolated polypeptide comprising a first and a second amino acid sequence, wherein
(a) the first amino acid sequence
   (a1) comprises in N- to C-terminus orientation the general amino acid sequence element
      GGR1-Linker-GGR2
      wherein GGR1 is a GG repeat sequence of the consensus sequence GGxGxDxUx, wherein each x can independently be any amino acid and U is independently a hydrophobic, large amino acid selected from F, V, A, Y, I, L and M;
      wherein GGR2 is a GG repeat sequence selected from any one of the amino acid sequences set forth in SEQ ID Nos. 1-50687, 1-40000, 1-30000, 1-20000, 1-10000, 1-8000, 1-6000, 1-5000, 1-4000, 1-3000, 1-2000, 1-1000, for example SEQ ID Nos. 1-500;
      wherein "Linker" is independently either a peptide bond or an amino acid sequence of 1 to 25 amino acids, preferably a peptide bond;
   (a2) wherein the first amino acid sequence is 18 to 300, preferably 30 to 200 amino acids in length; and
   (a3) wherein the complete first amino acid sequence and/or the amino acid sequence element GGR1-Linker-GGR2 is a non-natural sequence;
(b) the second amino acid sequence is at least one peptide or polypeptide of interest, and
(c) wherein the first and second amino acid sequence are heterologous to each other

In various embodiments, GGR2 is selected from any one of the amino acid sequences set forth in SEQ ID Nos. 2, 7, 19, 43, 110, 127, 525, and 597.

In various embodiments, the first amino acid sequence comprises at least 3, at least 4, at least 5, at least 6, at least 7 or at least 8 GG repeat sequences of the general consensus sequence GGxGxDxUx, wherein any third and further GG repeat sequence(s) are located C-terminal to the sequence element GGR1-Linker-GGR2 such that the first amino acid sequence optionally comprises the general structure, from N- to C-terminus,
(i) GGR1-Linker-GGR2-Linker-GGR3; or
(ii) GGR1-Linker-GGR2-Linker-GGR3-Linker-GGR4; or
(iii) GGR1-Linker-GGR2-Linker-GGR3-Linker-GGR4-Linker-GGR5; or
(iv) GGR1-Linker-GGR2-Linker-GGR3-Linker-GGR4-Linker-GGR5-Linker-GGR6; or
(v) GGR1-Linker-GGR2-Linker-GGR3-Linker-GGR4-Linker-GGR5-Linker-GGR6-Linker-GGR7; or
(vi) GGR1-Linker-GGR2-Linker-GGR3-Linker-GGR4-Linker-GGR5-Linker-GGR6-Linker-GGR7-Linker-GGR8
wherein GGR3, GGR4, GGR5, GGR6, GGR7 and GGR8 are each independently GG repeats of the consensus sequence GGxGxDxUx as defined above for GGR1, and "Linker" is also as defined above.

In various embodiments, the general sequence element GGR1-Linker-GGR2 or any of the general structures (i)-(vi) disclosed above is flanked by additional N- and/or C-terminal amino acid sequences, optionally each being up to 150 amino acids in length. The N-terminal flanking sequence may be 1 to 100 amino acids in length, preferably 1 to 80, 1 to 70, 5 to 60, 5 to 50, 5 to 40, 5 to 30, 8 to 20, 10 to 15 or 10 amino acids in length; and/or, in various embodiments, may comprise or consist of the amino acid sequence set forth in SEQ ID NO:50688. The C-terminal flanking sequence may be 10 to 150 amino acids in length, preferably 20 to 120, 30 to 120, 40 to 120, 50 to 120, 60 to 120, 70 to 120, 80 to 120, 90 to 120, 100 to 120 or 110 to 120 amino acids in length; and/or, in various embodiments, the C-terminal flanking sequence may comprise or consist of the amino acid sequence set forth in SEQ ID NO:50689.

The N- and/or C-terminal flanking sequences are scaffold sequences that may be derived from a natural occurring RTX protein, in which any one or more of the naturally-occurring GG repeat sequences have been replaced by the general sequence element GGR1-Linker-GGR2 or any of the general structures (i)-(vi) referred to above. The scaffold sequence may derived from any protein, such as RTX protein, in particular hemolysin A protein, preferably hemolysin A1 having the amino acid sequence set forth in SEQ ID NO:50690 or a variant or fragment thereof, more preferably the amino acid sequence as set forth in SEQ ID NO:50691 or a variant or fragment thereof, wherein the general sequence element GGR1-Linker-GGR2 or any of the general structures (i)-(vi) as disclosed above are inserted between positions 10 and 11 of SEQ ID NO:50691 using the positional numbering of SEQ ID NO:50691.

In various embodiments, U in the consensus sequence GGxGxDxUx is selected from F, V, A, I, L, M and Y, for example F, V, I, L, M and Y. The consensus sequence of the GG repeat sequences may thus be GGX¹GX²DX³UX⁴, wherein X¹, X², X³ and X⁴ may be any amino acid with the exception of P, preferably X¹ is selected from G, A, L, V, I, M, F, S, T, Q; Y, K, R, D, E, N, and Y, preferably from G, A, E, S, T, Q, L, R and D, more preferably from G, A, E, S and T; and/or X² is selected from N, D, A, G, S, H, T, E, M, R and H, preferably from N, D, A, S, amd H, more preferably from N, D, A, and S; and/or X³ is selected from A, K, V, L, I, F, M, R, Y, S, L, T, V, Q, N, D, E, and H, preferably from T, R, V, L, S, I, A, Y, Q, D and H, more preferably from T, R, V, L, S and I, even more preferably from T, R and V; and/or X⁴ is selected from W, K, R, Y, V, L, T, H, D, A, M, E, F, I, S, N and Q, preferably from V, L, I, F, S, R, N, Y and T.

In various embodiments, GGR1 and optionally any further GG repeat sequence are selected from the amino acid sequences set forth in SEQ ID Nos. 1-50687, preferably SEQ ID Nos. 1-40000, 1-30000, 1-20000, 1-10000, 1-8000, 1-6000, 1-5000, 1-4000, 1-3000, 1-2000, 1-1000 or 1-500.

In various embodiments,
(a) GGR2 is the amino acid set forth in SEQ ID NO:19 and GGR1 has the amino acid sequence set forth in any one of SEQ ID Nos. 2, 19, 218, 188, 1891, 57, 12347, 582, 8665, 5147, 10516, 2068, 3426, 17313, 3944, 48880, 192;
(b) GGR2 is the amino acid sequence set forth in SEQ ID NO:2 and GGR1 has the amino acid sequence set forth in SEQ ID NO:2;
(c) GGR2 is the amino acid sequence set forth in SEQ ID NO:7 and GGR1 has the amino acid sequence set forth in SEQ ID NO:57;
(d) GGR2 is the amino acid sequence set forth in SEQ ID NO:31 and GGR1 has the amino acid sequence set forth in SEQ ID NO:57;
(e) GGR2 is the amino acid sequence set forth in SEQ ID NO:32 and GGR1 has the amino acid sequence set forth in SEQ ID NO:28;
(f) GGR2 is the amino acid sequence set forth in SEQ ID NO:43 and GGR1 has the amino acid sequence set forth in SEQ ID NO:19;
(g) GGR2 is the amino acid sequence set forth in SEQ ID NO:110 and GGR1 has the amino acid sequence set forth in SEQ ID NO:19 or 192;
(h) GGR2 is the amino acid sequence set forth in SEQ ID NO:127 or 597 and GGR1 has the amino acid sequence set forth in SEQ ID NO:19; or
(i) GGR2 is the amino acid sequence set forth in SEQ ID NO:525 and GGR1 has the amino acid sequence set forth in SEQ ID NO:28.

In all these embodiments, "Linker" between GGR1 and GGR2 may be a peptide bond. In these embodiments, the first amino acid sequence may not comprise a further GG repeat sequence.

The isolated polypeptide typically is a fusion protein, with the second amino acid sequence optionally being 10 to 1000 amino acids in length, preferably 15 to 500 amino acids in length. In various embodiments, the second amino acid sequence is N-terminal or C-terminal to the first amino acid sequence, preferably C-terminal. The second amino acid sequence may be linked directly or via a linker sequence to the N- or C-terminal end of the first amino acid sequence, the linker sequence optionally being 1 to 30 amino acids in length and preferably comprising a protease recognition and cleavage site. The protease recognition and cleavage site may be a TEV protease recognition and cleavage site.

The isolated polypeptide may have a total length of up to 1000 amino acids, preferably up to800, up to 700, up to 600 or up to 500 amino acids, for example up to 350 amino acids.

In another aspect, the invention also relates to a nucleic acid, nucleic acid molecule or isolated nucleic acid molecule encoding the isolated polypeptide as described herein. In one aspect, said nucleic acid is part of a vector. One aspect thus features a (nucleic acid) vector comprising a nucleic acid molecule according to the invention. The vector may be an expression vector and may comprise additional nucleic acid sequences necessary to facilitate its function in a host cell. In various embodiments, the vector may be a plasmid.

One further aspect of the invention relates to a host cell comprising a nucleic acid molecule according to the invention or a vector according to the invention. The host cell may be a prokaryotic host cell, for example an *E.coli* cell.

In a still further aspect, the invention is directed to a method for the production of a polypeptide (isolated polypeptide) as described herein, comprising
(1) cultivating the host cell described herein under conditions that allow the expression of the polypeptide; and
(2) isolating the expressed polypeptide from the host cell; and, optionally,
(3) renaturing the isolated polypeptide.

The method may in various embodiments comprise recovering the expressed peptide or protein from the host cell in form of insoluble protein aggregates, in particular inclusion bodies (IBs). Such embodiments, in which the fusion protein is accumulated within the host cells, optionally in insoluble/denatured form, may be particularly advantageous alternatives of such methods.

In various embodiments, the methods may comprise a step of re-solubilizing the peptide/protein and/or reconstituting/refolding it under suitable conditions. Said step is also referred to herein as "renaturing" step. Such a step is described in more detail below in relation to methods for renaturing the isolated polypeptide.

In still another aspect, the present invention is therefore also directed to methods for renaturing the isolated polypeptide of the invention, wherein the method may comprise contacting, for example in form of re-solubilizing, the isolated polypeptide with a suitable medium for renaturation, typically an aqueous medium, optionally comprising earth alkaline metal ions, including but not limited to calcium ions. It has been found that such a medium may trigger conformational changes in the GG repeats (comprised in the first amino acid sequence) that in turn then facilitate refolding/renaturation of the fused heterologous peptide or polypeptide (second amino acid sequence). This effect is independent of the type of fused peptide or polypeptide in that the conformational change of the first amino acid sequence effects the renaturation of the second amino acid sequence.

In various embodiments of the methods of the invention, the host cell is a prokaryotic cell, for example an *E.coli* cell. In various embodiments, the expression is performed in minimal culture medium. In various embodiments, the recombinant peptide or protein is purified using a method selected from affinity chromatography, ion exchange chromatography, reverse phase chromatography, size exclusion chromatography, and combinations thereof; and/or the method comprises treatment of the recombinant peptide or protein with a protease suitable for cleavage of a protease cleavage site within the recombinant peptide or protein and, optionally, said treatment with a protease is followed by purification of the recombinant peptide or protein.

In still another aspect, the present invention also relates to the use of an isolated polypeptide of the invention for facilitating the production of a recombinant peptide or protein. Said recombinant peptide of protein may be part of the isolated polypeptide, which is expressed as a fusion protein. In such embodiments, the first amino acid sequences disclosed herein may be used to facilitate renaturation/refolding of said recombinant peptide or polypeptide. Said recombinant peptide or protein is identical with the peptide or polypeptide of interest as referred to herein and may thus consist of or be comprised in the second amino acid sequence disclosed herein. As stated above, said use is preferably directed to recombinant polypeptides expressed as fusion proteins in a suitable expression system. The present invention thus features the use of the isolated polypeptide of the present invention for expression and renaturation of the second amino acid sequence in form of a fusion protein, wherein the expression is in a recombinant host cell in form of inclusion bodies.

It is understood that all combinations of the above disclosed embodiments are also intended to fall within the scope of the present invention.

### DETAILED DESCRIPTION OF THE INVENTION

The terms used herein have, unless explicitly stated otherwise, the meanings as commonly understood in the art.

"At least one", as used herein, relates to one or more, in particular 1, 2, 3, 4, 5, 6, 7, 8, 9, 10 or more.

"Isolated" as used herein in relation to a molecule means that said molecule has been at least partially separated from other molecules it naturally associates with or other cellular components. "Isolated" may mean that the molecule has been purified to separate it from other molecules and components, such as other proteins and nucleic acids and cellular debris, in particular those that accompany it due to its recombinant production in host cells.

"Nucleic acid" as used herein includes all natural forms of nucleic acids, such as DNA and RNA. Preferably, the nucleic acid molecules of the invention are DNA.

The term "peptide" is used throughout the specification to designate a polymer of amino acid residues connected to each other by peptide bonds. A peptide according to the present invention may have 2-100 amino acid residues. The terms "protein" and "polypeptide" are used interchangeably throughout the specification to designate a polymer of amino acid residues connected to each other by peptide bonds. A protein or polypeptide according to the present invention has preferably more than 100 amino acid residues.

The term "an N-terminal fragment" relates to a peptide or protein sequence which is in comparison to a reference peptide or protein sequence C-terminally truncated, such that a contiguous amino acid polymer starting from the N-terminus of the peptide or protein remains. In some embodiments, such fragments may have a length of at least 30 amino acids, at least 50 amino acids or at least 70 amino acids.

The term "a C-terminal fragment" relates to a peptide or protein sequence which is in comparison to a reference peptide or protein sequence N-terminally truncated, such that a contiguous amino acid polymer starting from the C-terminus of the peptide or protein remains. In some embodiments, such fragments may have a length of at least 30 amino acids, at least 50 amino acids or at least 70 amino acids.

The term "fusion protein" as used herein concerns two or more peptides and proteins which are N- or C-terminally connected to each other, typically by (a) peptide bond(s), including via an amino acid/peptide linker sequence. Such fusion proteins may be encoded by two or more nucleic acid sequences which are operably fused to each other. In certain embodiments, a fusion protein refers to at least one peptide or protein of interest C-terminally or N-terminally fused to a first amino acid sequence according to the invention. In various embodiments, the peptide or protein of interest is fused to the C-terminus of the first amino acid sequence, optionally via a linker sequence.

"Stability", as used herein in relation to the polypeptides of the invention, primarily relates to resistance to proteolytic degradation, which is a commonly encountered issue, in particular in conditions of high cell density and/or high protein yields.

"Solubility", as used herein in relation to the polypeptides of the invention, primarily relates to solubility in the cytoplasm and/or culture medium so that the polypeptide can be successfully secreted and purified from the cultivation medium.

"Renaturation" and "renaturation efficiency", as used herein in relation to the polypeptides of the invention, primarily relates to the possibility to isolate the polypeptide in denatured/unfolded form, for example from insoluble protein aggregates, such as inclusion bodies, and induce its proper folding to the active, three-dimensional conformation. The efficiency may be given as the mass of successfully renatured protein relative to the total mass of all expressed protein, including insoluble protein, in percent. The refolding may be effected in special refolding buffers including earth alkaline metal ions, in particular calcium ions, to induce refolding of the first amino acid sequence, which in turn facilitates renaturation of the complete fusion construct into its functional conformation. "Renaturing" and "refolding" are used interchangeably herein and generally relate to the process of folding a given protein that is unfolded or denatured such that it adopts its functional three-dimensional conformation.

Generally, the skilled person understands that for putting the present invention into practice any nucleotide sequence described herein may comprise an additional start and/or stop codon or that a start and/or stop codon included in any of the sequences described herein may be deleted, depending on the nucleic acid construct used. The skilled person will base this decision, e.g., on whether a nucleic acid sequence comprised in the nucleic acid molecule of the present invention is to be translated and/or is to be translated as a fusion protein. In various embodiments, the isolated polypeptides of the invention additionally comprise the amino acid M on the N-terminus.

Determination of the sequence identity of nucleic acid or amino acid sequences can be done by a sequence alignment based on well-established and commonly used BLAST algorithms (See, e.g. Altschul, S.F., Gish, W., Miller, W., Myers, E.W. & Lipman, D.J. (1990) "Basic local alignment search tool." J. Mol. Biol. 215:403-410, and Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402). Such an alignment is based on aligning similar nucleotide or amino acid sequences stretches with each other. Another algorithm known in the art fo said purpose is the FASTA algorithm. Alignments, in particular multiple sequence comparisons, are typically done by using computer programs. Commonly used are the Clustal series (See, e.g., Chenna et al. (2003): Multiple sequence alignment with the Clustal series of programs. Nucleic Acid Research 31, 3497-3500), T-Coffee (See, e.g., Notredame et al. (2000): T-Coffee: A novel method for multiple sequence alignments. J. Mol. Biol. 302, 205-217) or programs based on these known programs or algorithms.

Also possible are sequence alignments using the computer program Vector NTI^{®} Suite 10.3 (Invitrogen Corporation, 1600 Faraday Avenue, Carlsbad, CA, USA) with the set standard parameters, with the AlignX module for sequence comparisons being based on the ClustalW. If not indicated otherwise, the sequence identity is determined using the BLAST algorithm.

Such a comparison also allows determination of the similarity of the compared sequences. Said similarity is typically expressed in percent identify, i.e. the portion of identical nucleotides/amino acids at the same or corresponding (in an alignment) sequence positions relative to the total number of the aligned nucleotides/amino acids. For example, if in an alignment 90 amino acids of a 100 aa long query sequence are identical to the amino acids in corresponding positions of a template sequence, the sequence identity is 90%. The broader term "homology" additionally considers conserved amino acid substitutions, i.e. amino acids that are similar in regard to their chemical properties, since those typically have similar chemical properties in a protein. Accordingly, such homology can be expressed in percent homology. If not indicated otherwise, sequence identity and sequence homology relate to the entire length of the aligned sequence.

In the context of the present invention, the feature that an amino acid position corresponds to a numerically defined position in a reference sequence means that the respective position correlates to the numerically defined position in said reference sequence in an alignment obtained as described above.

The hemolysin (Hly) secretion system is a protein secretion system which mostly occurs in gram-negative bacteria. This secretion system belongs to the family of type I secretion systems which transport their substrates in an ATP driven manner in a single step from the cytosol to the extracellular space without an intermediate station in the periplasm. The Hly secretion system comprises hemolysin B (HlyB) which represents an ATP-binding cassette (ABC) transporter, the membrane fusion protein hemolysin D (HlyD), and the universal outer membrane protein ToIC. The 110 kDa hemolytic toxin hemolysin A (HlyA) is a transport substrate of the Hly secretion system. On genetic level, the components necessary for hemolysin A-specific secretion are organized in an operon structure. The nucleic acid sequence encoding for hemolysin C (HlyC) also forms part of this operon but is not required for HlyA secretion through the Hly secretion system. HlyC catalyzes acylation of HlyA which renders HlyA hemolytic. HlyA is a protein which consists of 1024 amino acid residues and requires for its export via the Hly secretion system its C-terminus, comprising about 40-60 amino acids. Furthermore, HlyA is characterized in that it comprises N-terminally to the 40-60 C-terminal amino acids a domain comprising several glycine rich (GG) repeats, with the amino acid sequences of these GG repeats set forth in SEQ ID Nos. 2029, 747, 24, 29, 33, and 6001. The Hly secretion system and its T1 SS allocrit HlyA are used as a scaffold herein for the newly combined GG repeat sequences to demonstrate the functionality of the inventive concept. However, it is to be understood that the invention is not limited to these constructs derived from HlyA, since similar results have also be obtained with the GG repeat-containing sequences of other RTX proteins.

"GG repeat", "GG repeat sequence" and "glycine rich repeat", as used herein interchangeably, are the characteristic of the repeats in toxin (RTX) toxin family and have the consensus sequence disclosed herein. The glycine rich repeats bind Ca²⁺ which induces their folding. Hence, in absence of earth alkaline metal ions, in particular Ca²⁺, the domain comprising the glycine rich repeats is unstructured.

The present invention is based on the inventor's surprising finding that two or more naturally occurring GG repeat sequences may be newly combined and, optionally, inserted into a scaffold structure to yield non-naturally occurring amino acid sequences which when fused to a peptide/polypeptide of interest allow to efficiently renaturated the complete fusion protein including the peptide/polypeptide of interest due to the presence of the GG repeats, with the renaturation efficiency being significantly higher than that obtained with a natural RTX protein.

"Non-natural", as used herein in relation to the inventive first amino acid sequences and general sequence elements, means that said sequence does not naturally occur in an organism, but has been obtained by protein/peptide engineering. The first amino acid sequence is thus generally a recombinant sequence either by virtue of the GGR1-Linker-GGR2 element already being non-natural or by one or more of the GGR repeats and the remainder of the first amino acid sequence being heterologous to each other. In various embodiments, the GG repeat sequences and the additional flanking sequences of the scaffold, which are not GG repeat sequences, are heterologous to each other and do not occur in combination in natural proteins or peptides. This term also means that the first amino acid sequence is not a (continuous or discontinuous) fragment of a naturally occurring polypeptide, but rather differs in sequence. The sequence identity of the first amino acid sequence over its entire length is thus not 100% relative to any naturally occurring protein or fragment thereof, but always differs in a least 1 amino acid position.

The present invention is directed to such fusion proteins that comprise an artificial, non-natural "expression and renaturation tag" that comprises at least two GG repeats as defined herein that imparts the desired properties of increased renaturation efficiency to the fusion protein. This is achieved by using said tag sequence as the first amino acid sequence, while the peptide(s) and/or polypeptide(s) of interest, i.e. the cargo that is to be produced, is the second amino acid sequence. These fusion proteins are also more generally referred to herein as polypeptides that are isolated after production. These fusion proteins and isolated polypeptides are artificial constructs, since the first and second amino acid sequences are typically heterologous to each other and/or the fusion construct has been genetically engineered.

As it is desirable to have maximum yields of the peptide or protein of interest, the first amino acid sequences of the invention are preferably as short as possible, generally with a maximum length of about 250 amino acids, typically about 200 amino acids or less. Herein constructs are used and exemplified wherein the first amino acid sequence has a length of about 150 amino acids. Together with optional linker and protease cleavage sites, the total length of the part of the fusion protein not including the peptide or protein of interest is typically limited to about 250 amino acids or less. In various embodiments, it may be desirable to use first amino acid sequences significantly short than the upper limit of 200 amino acids, such as up to 190, up to 180, up to 170, up to 160, or up to 150 amino acids in length. In various embodiments, the first amino acid sequence is long enough to accommodate 4, 5, or 6 GG repeats, i.e. is at least 36, at least 45 or at least 54 amino acids in length.

The first amino acid sequence comprises a first GG repeat sequence, herein referred to as GGR1, and a second GG repeat sequence, herein referred to as GGR2. The terms "first" and "second" as used in this context, as well as any further numbering terms, such as "third" etc., relate to the position of the respective GG repeat in the N- to C-terminal direction. The GG repeat closest to the N-terminus is thus always referred to as first GG repeat, the one following in C-terminal direction is the second GG repeat and so on.

The first GG repeat, i.e. GGR1, has the general consensus sequence GGxGxDxUx, wherein x can be any amino acid and U is a hydrophobic, large amino acid selected from F, V, A, I, L, M and Y. In various embodiments, the U in the consensus sequence is selected from F, V, I, L, M and Y or F, I, L, M and Y. In various embodiments, x is typically selected from the 20 proteinogenic amino acids G, A, V, L, I, F, C, M, P, R, K, H, N, Q, D, E, S, T, W, and Y. If not indicated otherwise, it is generally preferred that any amino acid not specifically defined is selected from these 20 proteinogenic amino acids. In various embodiments, x may be any one of the above amino acids with the exception of proline. In even more specific embodiments, the consensus sequence may be GGX¹GX²DX³UX⁴, wherein X¹, X², X³ and X⁴ may be any amino acid with the exception of P. In various embodiments, X¹ is selected from G, A, L, V, I, M, F, S, T, Q; Y, K, R, D, E, N, and Y, for example from G, A, E, S, T, Q, L, R and D, or from G, A, E, S and T. In various embodiments, X² is selected from N, D, A, G, S, H, T, E, M, R and H, for example from N, D, A, S, and H, or from N, D, A, and S. In various embodiments, X³ is selected from A, K, V, L, I, F, M, R, Y, S, L, T, V, Q, N, D, E, and H, for example from T, R, V, L, S, I, A, Y, Q, D and H, or from T, R, V, L, S and I, or from T, R and V. In various embodiments, X⁴ is selected from W, K, R, Y, V, L, T, H, D, A, M, E, F, I, S, N and Q, for example from V, L, I, F, S, R, N, Y and T. These embodiments of X¹-X⁴ may be combined with each other.

The same definition of the GG repeat sequence also applies to the (optional) third and any subsequent GG repeat.

In contrast to GGR1 and optionally GGR3 and any further C-terminal GG repeat sequence, the second GG repeat sequence is selected from the amino acid sequences set forth in SEQ ID Nos. 1-50687, 1-40000, 1-30000, 1-20000, 1-10000, 1-8000, 1-6000, 1-5000, 1-4000, 1-3000, 1-2000, 1-1000, for example SEQ ID Nos. 1-500. It has been surprisingly found by the inventors that the second GG repeat sequence more or less dominates the renaturation efficiency of the first amino acid sequence in a sequence specific matter. This means that certain GG repeat sequences if used as second GG repeats, GGR2, can surprisingly influence the renaturation of the fusion protein to a greater extent than the same sequence in another position, such as GGR1. With respect to the specific sequences useful as GGR2, it has been found that efficiency in increasing renaturation roughly correlates with the frequency with which this sequence occurs in nature. In other words, it has been found that those GG repeats that are more frequently encountered in natural proteins (as determined on protein databank analyses), irrespective of their position in these proteins, work particularly well if used as second GG repeats in the claimed fusion proteins. In contrast, while the first GG repeat is a necessary structural element for effecting renaturation of the fusion protein, its sequence is much less relevant. This means that the presence of any GG repeat sequence as GGR1 is sufficient for good renaturation, while GGR2 needs to be selected from those GG repeat sequences that occur with higher frequency in nature than others. For this purpose, the inventors have compiled 50687 different GG repeat sequences from publicly available protein and genomic databanks and have ranked them according to the frequency in which they occur in nature, with sequence 1 being the most frequent and 50687 being the least frequent. These sequences are set forth in the respective order in SEQ ID Nos. 1 to 50687. It has been found that the more frequent sequences provide for better renaturation than the less frequent sequences when used as GGR2. In contrast to this finding, the frequency of GGR1 does not appear to play a role. In accordance with this finding, the second GG repeat sequence is preferably defined herein to be selected from the 1000 most frequently encountered GG repeats, i.e. SEQ ID Nos. 1-1000, preferably from the most frequent 900 sequences, i.e. SEQ ID Nos. 1-900. The subgroup may be further limited such that GGR2 is selected from SEQ ID Nos. 1-800, 1-700, 1-600 or 1-500. Some GGR2 sequences that have been found to work particularly well are those of SEQ ID Nos. 2, 7, 19, 43, 110, 127, 525, and 597, with those of SEQ ID Nos. 19 and 110 being particularly preferred, with that of SEQ ID NO:19 being the most preferred sequence for GGR2.

In various embodiments, the first amino acid sequence may comprise more than 2 GG repeat sequences, such as at least 3, at least 4, at least 5, at least 6, at least 7 or at least 8 GG repeat sequences of the general consensus sequence GGxGxDxUx. However, while it has been found that in some instances a third and fourth GG repeat may further increase renaturation efficiency, although not to the same extent as the step from one to two GG repeats, adding a fifth and further GG repeat sequence appears to only incrementally influence renaturation efficiency in most cases. As the use of only two GG repeat sequences as reported herein has been found to perform significantly better than a natural sequence of 3 GG repeats and one GG repeat-like sequence and it is generally desirable to keep the first amino acid as short as possible, in various embodiments, it is preferred to use 2, 3 or 4 GG repeats.

In the first amino acid sequences of the invention, the GG repeats may be arranged directly adjacent to each other, i.e. directly connected by peptide bonds, or they may be separated by linkers. If there are more than 2 GG repeats both options can also be mixed in that some GG repeats are directly adjacent, while others are connected via linkers. These linker sequences may be amino acid sequences of 1 to 25 amino acids, preferably 1 to 20 or 1 to 15 or 1 to 10 or 1 to 9 or 1 to 8 or 1 to 7 or 1 to 6 or 1 to 5 or 1 to 4 or 1 to 3 or 1-2 amino acids in length. These are not limited with respect to their sequences (aside from not containing a GG repeat sequence), but, in various embodiments, it may be preferred to keep these linking sequences short, for example of only 12, 11, 10, 9, 8, 7, 6, 5, 4, 3, 2 or 1 amino acid in length. If two or more GG repeat sequences are contained in the first amino acid sequence it may be preferred that at least two, or depending on the number present, at least three, at least 4, at least 5, at least 6 or all of them are directly linked to each other by a peptide bond. It has been found, for example, that in some embodiments constructs where the first amino acid sequence comprises GG repeat sequences, with these sequences directly linked to each other, perform particularly well so that the presence of linker sequences may be unnecessary in most cases..

In various embodiments, the first amino acid sequence therefore comprises the general structure, from N- to C-terminus:
GGR1-Linker-GGR2.

This sequence element may be further expanded to be:
(i) GGR1-Linker-GGR2-Linker-GGR3; or
(ii) GGR1-Linker-GGR2-Linker-GGR3-Linker-GGR4; or
(iii) GGR1-Linker-GGR2-Linker-GGR3-Linker-GGR4-Linker-GGR5; or
(iv) GGR1-Linker-GGR2-Linker-GGR3-Linker-GGR4-Linker-GGR5-Linker-GGR6
(v) GGR1-Linker-GGR2-Linker-GGR3-Linker-GGR4-Linker-GGR5-Linker-GGR6-Linker-GGR7
(vi) GGR1-Linker-GGR2-Linker-GGR3-Linker-GGR4-Linker-GGR5-Linker-GGR6-Linker-GGR7-Linker-GGR8.

In these embodiments, GGR1, GGR3, GGR4, GGR5, GGR6, GGR7 and GGR8 are GG repeats of the consensus sequence GGxGxDxUx, as defined above, and wherein each "Linker" is independently either a peptide bond or an amino acid sequence of 1 to 25 amino acids, preferably 1 to 20 or 1 to 15 amino acids in length. The "Linker" does not comprise a GG repeat sequence. As disclosed above, it may be preferred that at least one "Linker" is a peptide bond. It is understood that also constructs with more than 8 GG repeats are covered, with the ninth and further GG repeat also linked via a "Linker" as herein defined.

In various embodiments, the GG repeat used in the first amino acid sequences as GGR1, GGR3, GGR4, GGR5, GGR6, GGR7, GGR8 or any further GGR (with the exception of GGR2) are selected from the sequences set forth in SEQ ID Nos. 1-50687, for example SEQ ID Nos. 1-40000, 1-30000, 1-20000, 1-10000, 1-5000, 1-4000, 1-3000, 1-2000, 1-1000, 1-500 or any one of SEQ ID Nos. 2, 19, 218, 188, 1891, 57, 12347, 582, 8665, 5147, 10516, 2068, 3426, 17313, 3944, 48880, and 192.

While the GG repeat sequences disclosed may be combined in any possible manner covered by the present disclosure, in various embodiments, the GG repeat sequence element in the first amino acid sequence in the isolated polypeptide satisfies the following:
(a) GGR2 is the amino acid set forth in SEQ ID NO:19 and GGR1 has the amino acid sequence set forth in any one of SEQ ID Nos. 2, 19, 218, 188, 1891, 57, 12347, 582, 8665, 5147, 10516, 2068, 3426, 17313, 3944, 48880, 192;
(b) GGR2 is the amino acid sequence set forth in SEQ ID NO:2 and GGR1 has the amino acid sequence set forth in SEQ ID NO:2;
(c) GGR2 is the amino acid sequence set forth in SEQ ID NO:7 and GGR1 has the amino acid sequence set forth in SEQ ID NO:57;
(d) GGR2 is the amino acid sequence set forth in SEQ ID NO:31 and GGR1 has the amino acid sequence set forth in SEQ ID NO:57;
(e) GGR2 is the amino acid sequence set forth in SEQ ID NO:32 and GGR1 has the amino acid sequence set forth in SEQ ID NO:28;
(f) GGR2 is the amino acid sequence set forth in SEQ ID NO:43 and GGR1 has the amino acid sequence set forth in SEQ ID NO:19;
(g) GGR2 is the amino acid sequence set forth in SEQ ID NO:110 and GGR1 has the amino acid sequence set forth in SEQ ID NO:19 or 192;
(h) GGR2 is the amino acid sequence set forth in SEQ ID NO:127 or 597 and GGR1 has the amino acid sequence set forth in SEQ ID NO:19; or
(i) GGR2 is the amino acid sequence set forth in SEQ ID NO:525 and GGR1 has the amino acid sequence set forth in SEQ ID NO:28.

The GG repeat sequence element may also be of structure (i) as disclosed herein above, wherein GGR1 is SEQ ID NO:19 or 192, GGR2 is SEQ ID NO:192 and GGR3 is SEQ ID NO:110.

The GG repeat structure may also be of structure (ii) as disclosed herein above, wherein GGR1 is SEQ ID NO:19 or 192, GGR2 is SEQ ID NO:192, GGR3 is SEQ ID NO:192, and GGR4 is SEQ ID NO:110.

The GG repeat structure may also be of structure (v) as disclosed herein above, wherein GGR1 is SEQ ID NO:19, GGR2 is SEQ ID NO:192, GGR3 is SEQ ID NO:19, GGR4, GGR5, GGR6 are all SEQ ID NO:192, and GGR7 is SEQ ID NO:19.

In all the afore-mentioned embodiments, the linker may be as defined herein, but is preferably a peptide bond so that all GG repeats are directly adjacent to each other.

The GG repeat sequence elements of the first amino acid sequence disclosed herein and more particularly the general structures given above, i.e. GGR1-Linker-GGR2 and (i) to (vi) as well as the specific embodiments disclosed above, may be flanked by additional N- and/or C-terminal amino acid sequences. These do not contain any GG repeat sequences and are each typically up to 200, preferably up to 150 amino acids in length. In various embodiments, these flanking sequences are of a length that the preferred length of the first amino acid sequence disclosed above is not exceeded.

In various embodiments, the N-terminal flanking sequence is 1 to 100 amino acids in length, preferably 1 to 80, 1 to 70, 5 to 60, 5 to 50, 5 to 40, 5 to 30, 8 to 20, 10 to 15 or 10 amino acids in length. In can be preferred that the N-terminal flanking sequence is as short as possible. In various embodiments, the N-terminal flanking sequence comprises, consists essentially of (i.e. does not contain more than 1-10 additional amino acids) or consists of the amino acid sequence GNSLAKNVLF (SEQ ID NO:50688). This sequence is derived from HlyA and artificially combined with the GG repeat elements disclosed herein.

In various embodiments, the C-terminal flanking sequence is 10 to 150 amino acids in length, preferably 20 to 120, 30 to 120, 40 to 120, 50 to 120, 60 to 120, 70 to 120, 80 to 120, 90 to 120, 100 to 120 or 110 to 120 amino acids in length. In various embodiments, the C-terminal flanking sequence is longer than the N-terminal flanking sequence. In various embodiments, the C-terminal flanking sequence comprises, consists essentially of (i.e. does not contain more than 1 to 50 additional amino acids) or consist of the amino acid sequence set forth in SEQ ID NO:50689. This sequence is derived from HlyA and artificially combined with the GG repeat elements disclosed herein.

In various embodiments, the N- and/or C-terminal flanking sequences are scaffold sequences derived from a natural occurring RTX protein, in which any one or more of the naturally-occurring GG repeat sequences have been replaced by the general sequence element GGR1-Linker-GGR2 or any of the general structures (i)-(vi). It may be that the complete GG repeat cassette in the respective RTX protein is replaced by the GG repeat elements disclosed herein and that the sequences N- and C-terminal to this cassette in the natural protein are N- and/or C-terminally truncated such that only the directly adjacent flanking sequences remain. These N- and C-terminal flanking sequences in combination form a scaffold in which the GG repeat sequence elements disclosed herein can be inserted.

In various embodiments, the flanking sequences may be fragments of a naturally occurring RTX protein, such as those disclosed herein below. Such fragments are typically at least 30 amino acids in length with a maximum length of 200 amino acids and do not comprise GG repeat sequences. In various embodiments, said fragments do not retain a functional, typical C-terminal, secretion signal, e.g. at least the C-terminal 40, preferably 50 or 60 amino acids (i.e. typically the 40 to 60 C-terminal amino acids of the native, naturally occurring protein) are not part of the fragment. The fragments are thus typically N-terminal fragments that may additionally lack most of the N-terminus and retain only the amino acid sequences in direct vicinity of the replaced GG repeats. These retained sequences adjacent to the core GG repeat sequence are referred to herein as "flanking sequences".

These RTX proteins may be allocrits of a T1 SS and may be selected from the group consisting of, without limitation, HlyA, CyaA, EhxA, LktA, PILktA, PasA, PvxA, MmxA, LtxA, ApxIA, ApxIIA, ApxIIIA, ApxIVA, Apxl, Apx11, AqxA, VcRtxA, VvRtxA, MbxA, RTX cytotoxin, RtxL1, RtxL2, FrhA, LipA, TliA, PrtA, PrtSM, PrtG, PrtB, PrtC, AprA, AprX, ZapA, ZapE, Sap, HasA, colicin V, LapA, ORF, RzcA, RtxA, XF2407, XF2759, RzcA, RsaA, Crs, CsxA, CsxB, SlaA, SwmA, S111951, NodO, PlyA, PlyB, FrpA, FrpC, RTX1, RTX3, RTX7, RTX8, RTX9, RTX12, RTX14, RTX16, RTX18, RTX19, RTX20, RTX21, RTX22, RTX24, RTX26, RTX28, RTX29, RTX30, RTX31, RTX32, and RTX33. The amino acid sequences of the recited RTX proteins are available in the Uni Prot database under the following accession numbers:
RTX1 (SCP-domain containing protein, *Aromatoleum aromaticum strain EbN1;* NCBI Reference WP_011239843.1; UniProt Accession No. Q5NXL6),
RTX3 (Serralysin (*Caulobacter sp.* strain K31); UniProt Accession No. B0T558),
RTX7 (uncharacterized protein of *Pelobacter propionicus*; UniProt Accession No. A1ARK7; NCBI Reference WP_011736233.1),
RTX8 (Metallopeptidase AprX (*Pseudomonas fluorescens strain ATCC BAA-477*/ *NRRL B-23932* /*Pf-*5); UniProt Accession No. Q4KDU3; NCBI Reference WP_011060780.1),,
RTX9 (glycosyl hydrolase family 16, hemolysin-type calcium-binding repeat protein (*Rhodobacter sphaeroides strain ATCC 17023* / *DSM 158* / *JCM 6121*); UniProt Accession No. Q3J0J9; NCBI Reference WP_011338282.1),
RTX12 (endonuclease/exonuclease/phosphatase (*Caulobacter sp.* strain K31); UniProt Accession No. B0T829; NCBI Reference WP_012287684.1),
RTX14 (Glycoside hydrolase family 16 (*Caulobacter sp.* strain K31); UniProt Accession No. B0T438),
RTX16 (lipase class 3 (*Chlorobium phaeobacteroides strain DSM266*); UniProt Accession No. A1BIU7; NCBI Reference WP_011746110.1),
RTX18 (animal haem peroxidase (*Leptotrix cholodnii srain ATCC 51168* / *LMG 8142* / *SP-6*); UniProt Accession No. B1Y442; NCBI Reference WP_012347322.1),
RTX19 (putative outer membrane adhesin like protein (*Magnetococcus marinus strain ATCC BAA-1437* /*JCM 17883* / *MC-1*); UniProt Accession No. A0L6L9; ENA Reference ABK43612.1),
RTX20 (putative outer membrane adhesin like protein (*Magnetococcus marinus strain ATCC BAA-1437* /*JCM 17883* / *MC-1*); UniProt Accession No. A0LDY6; NCBI Reference WP_011715232.1),
RTX21 (Glycerophosphoryl diester phosphodiesterase (*Methylobacterium nodulans* (*strain LMG 21967* / *CNCM I-2342* / *ORS 2060*); UniProt Accession No. B8ICD8; EMBL Reference ACL55526.1),
RTX22 (5' nucleotidase domain protein (*Methylobacterium sp. strain 4-64*); UniProt Accession No. B0UQ63; NCBI Reference WP_012331605.1),
RTX24 (polyurethanase A (*Pseudomonas fluorescens strain ATCC BAA-477* / *NRRL* B 23932 / *Pf-5*); UniProt Accession No. Q4KBS6; NCBI Reference WP_011061486.1),
RTX26 (extracellular alkaline metalloprotease AprA (*Pseudomonas fluorescens strain ATCC BAA-477*/ *NRRL B-23932* / *Pf-5*); UniProt Accession No. Q4KBR8; NCBI Reference WP_011061494.1),
RTX28 (hemolysin type calcium-binding protein (*Rhodobacter sphaeroides strain ATCC 17023* /*DSM 158* / *JCM 6121);* UniProt Accession No. Q3IVG4; NCBI Reference WP_011331290.1),
RTX29 (neutral zinc metallopeptidase (*Rhodobacter sphaeroides strain ATCC 17023* / *DSM 158* / *JCM* 6121); UniProt Accession No. Q3J1D3; NCBI Reference WP_011338088.1),
RTX30 (glycoside hydrolase family 16 (*Rhodobacter sphaeroides strain ATCC 17029* / *ATH 2.4.9);* UniProt Accession No. A3PLQ2; NCBI Reference WP_011841485.1),
RTX31 (peptidase domain protein (*Sinorhizobium medicae strain WSM419*); UniProt Accession No. A6UK74; NCBI Reference WP_011970163.1),
RTX32 (Serralysin (*Sinorhizobium medicae strain WSM419*); UniProt Accession No. A6UK83; NCBI Reference WP_011970172.1), and
RTX33 (hemolysin type calcium-binding region (*Verminephrobacter eiseniae strain EF01-2*); UniProt Accession No. A1WL15; NCBI Reference WP_011810323.1).

As disclosed herein, neither are these proteins used as such as the first amino acid sequence, nor are fragments thereof that contain the natural GG repeat sequences used as the first amino acid sequence. Rather only the flanking sequences of these proteins are used to create a scaffold sequence for the newly developed GG repeat elements disclosed herein and combined such as to form a non-natural first amino acid sequence that does not share 100% sequence identity with any natural protein sequence over its entire length (meaning the GG repeat element plus optional flanking sequences). In various embodiments, the sequence identity of such a first amino acid sequence to a natural protein is 99% or less, 98% or less, 97% or less, 96%or less, 95% or less, 94% or less, 93% or less, 92% or less, 91% or less, 90% or less, 89% or less, 88% or less, 87% or less, 86% or less, or 85% or less,

The scaffold sequence may be derived from hemolysin A protein, preferably hemolysin A1 having the amino acid sequence set forth in SEQ ID NO:50690 or a variant or fragment thereof, more preferably the amino acid sequence as set forth in SEQ ID NO:50691 or a variant or fragment thereof, wherein the general sequence element GGR1-Linker-GGR2 or any of the general structures (i)-(vi) according to claim 3 are inserted between positions 10 and 11 using the positional numbering of SEQ ID NO:50691.

In alternative embodiments, the scaffold sequence formed by the flanking sequences can be derived from any other RTX protein, such as RTX1, RTX9, RTX12, RTX18, RTX21, RTX22, RTX30, RTX33, RTX28, RTX24, RTX19, RTX16, RTX26, RTX3, RTX20, RTX31, RTX14, RTX32 and RTX29.

In various exemplary embodiments, the flanking sequences may be derived from a known C-terminal fragment of HlyA, namely HlyA1 (SEQ ID NO:50690). Said known fragment comprises 3 GG repeats These GG repeats are deleted in such scaffolds and replaced by a GG repeat element that differs from the natural, deleted GG repeat element. In various embodiments, the scaffold is a further shortened version of HlyA1 (with deleted GG repeat cassette, i.e. positions 11 to 46 of SEQ ID NO:50690 being deleted) obtained by any one or more of an N-terminal truncation, a C-terminal truncation or a deletion of one or more amino acids, in particular a C-terminal truncation. In such embodiments, the remaining length of the scaffold may be at least 50 amino acids in length, preferably at least 60, 70, 80, 90, 100, 110, 120, 130, 140, 150, 160, 170, or 180 amino acids long. In various embodiments, the scaffold fragment of SEQ ID NO:50690 has a C-terminal truncation, for example up to 30, up to 35, up to 40, up to 45, up to 50, up to 55 or up to 60 amino acids. In various embodiments, the scaffold sequence lacks the GG repeat cassette of HlyA1, i.e. amino acids 11-46, and the 54 C-terminal amino acids, i.e. positions 165-218, using the positional number of SEQ ID NO:50690. This yields the scaffold sequence set forth in SEQ ID NO:50691. In this scaffold sequence, which is devoid of GG repeats, the GG repeat element of choice is then inserted between amino acids 10 and 11, using the positional numbering of SEQ ID NO:50691.

The invention further comprises, as flanking sequences, variants of all the afore-mentioned fragments of RTX proteins. These variants may have over their entire length, at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95%, at least 96 %, at least 97 %, at least 97.5%, at least 98 %, at least 98.5 %, at least 99% or 99.5% sequence identity with the respective amino acid sequence. In various embodiments, the variant is of the same length as the respective reference sequence. In other embodiments, it is a further shortened fragment thereof that may be obtainable by deletions/truncations. As noted, this variability is only given for the so-called flanking sequences that are N-terminal to the most N-terminal GG repeat and C-terminal to the most C-terminal GG repeat.

The first amino acid sequence of the present invention is, in various embodiments, 18 to 300 amino acids in length, for example 30 to 200. The lower limit may be 20, 25, 30, 35, 40, 45, 50, 55, 60, 65, 70, 75, 80, 85, 90, 95 or 100 amino acids in length. As disclosed above, it may be preferred that the first amino acid sequence is as short as possible, as long as its beneficial influence on expression levels and/or yields is not impaired and it retains sufficiently high solubility and/or proteolytic stability. Generally, the skilled person will know how to find a balance between a sequence that is as short as possible while still providing for the desired expression levels/yields within the scope defined by the present invention.

In various embodiments, if the first amino acid sequence has a scaffold derived from SEQ ID NO:50690, the first amino acid sequence does not comprise any one of the full amino acid sequences of SEQ ID Nos. 46-50 as set forth in WO 2013/057312 A1. In various other embodiments, the first amino acid sequence does not comprise any one of the full amino acid sequences of SEQ ID Nos. 33, 34, 36 of EP 2 583 975 A1, as well as SEQ ID Nos. 4 and 7 of WO 2006/036406 A2 and EPOP:A12703 (200 aa long C-terminal fragment of HlyA) of WO 8706953 A1. In various embodiments, the isolated polypeptide is not that of SEQ ID NO: 28, 30, 32, 34 or 36 of EP 2792686 A1. In various embodiments, the first amino acid sequence does not comprise the first amino acid sequence as disclosed in PCT/EP2021/056948 or EP20163961 or EP20168779, such as any one of SEQ ID Nos. 1, 8-26, 44-63, and 65-71 of PCT/EP2021/056948. These known sequences are excluded as being the first amino acid sequence by the definitions given herein, as they are fragments of the natural HlyA protein and thus excluded by the definition that the first amino acid sequence is a "non-natural" sequence (that differs from any natural protein by at least one amino acid substitution, preferably more).

The first amino acid sequence, as disclosed herein, is generally of the structure
"N-terminal flanking sequence" - "GGR repeat element" - "C-terminal flanking sequence"
with the GG repeat element being GGR1-Linker-GGR2 or any one of (i) to (vi) disclosed above. The first amino acid sequence may consist of such a structure.

In various embodiments, the first amino acid sequence does not comprise a secretion signal, i.e. an amino acid sequence that facilitates secretion from the host cell into the medium. This may mean that the first amino acid sequence does not facilitate secretion of the fusion protein to a significant degree, which may mean that at least 50 % or more of the expressed fusion protein remain inside of the cell, preferably as inclusion bodies, and are not secreted to the surrounding medium. This percentage may be higher, such as 60%, 70%, 80%, 90% or more.

The isolated polypeptide comprises a second amino acid sequence N-terminal or C-terminal to the first amino acid sequence, wherein the second amino acid sequence is at least one peptide or polypeptide of interest. The second amino acid sequence may be 5 to 1500 amino acids in length, preferably 10, 12 or 15 to 1000, to 800, to 700, to 600, to 500, to 400, to 300, to 200, to 180, to 150, to 120 or to 100 amino acids in length. The terms "second amino acid sequence" and the terms "peptide of interest" and "polypeptide/protein of interest" are used interchangeably herein. "Peptides of interest" are typically oligopeptides with a length of up to 100 amino acids, typically 3 to 80 or 5 to 70 amino acids. "Polypeptides/proteins of interest" typically comprise more than 100 amino acids, with the upper limit as defined above. The (poly)peptides of interest are preferably not the (poly)peptides from which either the GG repeats and/or the flanking sequences of the first amino acid sequence are derived.

In various embodiments, the peptide or protein of interest may comprise two or more naturally occurring peptides or proteins, the two or more peptides or proteins may be separated by protease cleavage sites. This also includes embodiments, where the same peptide or protein is included multiple times in said second amino acid sequence. This then allows production of higher amounts of the respective peptide or protein of interest. In other embodiments, the peptide or protein of interest is only a single peptide or protein.

Generally, any peptide or protein may be chosen as protein of interest. In certain embodiments, the protein of interest is a protein, which does not form a homo-dimer or homo-multimer. The protein of interest may also be a peptide or protein which is a subunit of a larger peptide or protein complex. Such a peptide or protein may be isolated after expression and be suitable for an *in vitro* reconstitution of the multi peptide or protein complex. In certain embodiments, the protein or peptide of interest is a protein or peptide having less than 1000, less than 800, less than 700, less than 600, less than 500, less than 400, less than 300 amino acid residues, for example less than 200 amino acids or less than 150 amino acids. If these peptides comprise pre- and/or pro- sequences in their native state after translation the nucleic acid sequence encoding for the peptide of interest may be engineered to be limited to the sequence encoding the mature peptide. One exemplary peptide is insulin, e.g., human insulin. The expression of over-expressed peptides and proteins as inclusion bodies is especially advantageous where the peptide or protein is harmful to the host cell. For this reason, the present invention is particularly advantageous for expression of lipases and proteases which are known to be toxic to the host cell and thus the expression of these proteins by the inventive systems and methods represents a specific embodiment of the present invention.

In various embodiments, the peptide or protein of interest is an enzyme. The International Union of Biochemistry and Molecular Biology has developed a nomenclature for enzymes, the EC numbers; each enzyme is described by a sequence of four numbers preceded by "EC". The first number broadly classifies the enzyme based on its mechanism. The complete nomenclature can be browsed at http://www.chem.qmul.ac.uk/iubmb/enzyme/.

Accordingly, a peptide or protein of interest according to the present invention may be chosen from any of the classes EC 1 (Oxidoreductases), EC 2 (Transferases), EC 3 (Hydrolases), EC 4 (Lyases), EC 5 (Isomerases), and EC 6 (Ligases), and the subclasses thereof.

In certain embodiments, the peptide or protein of interest is cofactor dependent or harbors a prosthetic group. For expression of such peptides or proteins, in some embodiments, the corresponding cofactor or prosthetic group may be added to the culture medium during expression.

In certain cases, the peptide or protein of interest is a dehydrogenase or an oxidase. In case the peptide or protein of interest is a dehydrogenase, in some embodiments, the peptide or protein of interest is chosen from the group consisting of alcohol dehydrogenases, glutamate dehydrogenases, lactate dehydrogenases, cellobiose dehydrogenases, formate dehydrogenases, and aldehydes dehydrogenases. In case the peptide or protein of interest is an oxidase, in some embodiments, the peptide or protein of interest is chosen from the group consisting of cytochrome P450 oxidoreductases, in particular P450 BM3 and mutants thereof, peroxidases, monooxygenases, hydrogenases, monoamine oxidases, aldehydes oxidases, xanthin oxidases, amino acid oxidases, and NADH oxidases.

In further embodiments, the peptide or protein of interest is a transaminase or a kinase. In case the peptide or protein of interest is a transaminase, in some embodiments, the peptide or protein of interest is chosen from the group consisting of alanine aminotransferases, aspartate aminotransferases, glutamate-oxaloacetic transaminases, histidinol-phosphate transaminases, and histidinol-pyruvate transaminases. In various embodiments, if the peptide or protein of interest is a kinase, the peptide or protein of interest is chosen from the group consisting of nucleoside diphosphate kinases, nucleoside monophosphate kinases, pyruvate kinase, and glucokinases.ln some embodiments, if the peptide or protein of interest is a hydrolase, the peptide or protein of interest is chosen from the group consisting of lipases, amylases, proteases, cellulases, nitrile hydrolases, halogenases, phospholipases, and esterases.

In certain embodiments, if the peptide or protein of interest is a lyase, the peptide or protein of interest is chosen from the group consisting of aldolases, e.g., hydroxynitrile lyases, thiamine-dependent enzymes, e.g., benzaldehyde lyases, and pyruvate decarboxylases. In various embodiments, if the peptide or protein of interest is an isomerase, the peptide or protein of interest is chosen from the group consisting of isomerases and mutases.

In some embodiments, if the peptide or protein of interest is a ligase, the peptide or protein of interest may be a DNA ligase.

In certain embodiments, the peptide or protein of interest may be an antibody. This may include a complete immunoglobulin or fragment thereof, which immunoglobulins include the various classes and isotypes, such as IgA, IgD, IgE, IgGI, IgG2a, IgG2b and IgG3, IgM, etc. Fragments thereof may include Fab, Fv and F(ab')2, Fab', the variable domain of the light chain (VL) or the variable domain of the heavy chain (VH) and related fragments, such as nanobodies, and the like.

Also contemplated herein are therapeutically active peptides and proteins of interest, e.g., cytokines.

Thus, in certain embodiments the peptide or protein of interest is selected from the group consisting cytokines, in particular human or murine interferons, interleukins (IL-1, IL-2, IL-3, IL-4; IL-5; IL-6; IL-7; IL-8; IL-9; IL-10; IL-11; IL-12; IL-13; IL-14; IL-15; IL-16; and IL-17), colony-stimulating factors, necrosis factors, e.g., tumor necrosis factor, such as TNF alpha, and growth factors, such as transforming growth factor beta family members, such as TGF-beta1; TGF-beta2 and TGF-beta3.

In some embodiments, if the peptide or protein of interest is an interferon, the peptide or protein of interest may be selected from the group consisting of interferon alpha, e.g., alpha-1, alpha-2, alpha-2a, and alpha-2b, alpha-2, alpha-16, alpha 21, beta, e.g., beta-1, beta-1a, and beta-1b, or gamma.

In further embodiments, the peptide or protein of interest is an antimicrobial peptide, in particular a peptide selected from the group consisting of bacteriocines and lantibiotics, e.g., nisin, cathelicidins, defensins, and saposins.

Also disclosed herein are peptides or proteins of interest which are therapeutically active peptides or proteins. In certain embodiments, the peptide or protein of interest is a therapeutically active peptide. In some embodiments, a therapeutically active peptide may be selected from the group consisting of Fuzeon/T20, human calcitonin, salmon calcitonin, human corticotropin release factor, Mab40, Mab42, peptides associated with Alzheimer's disease, exenatide, Tesamorelin, Teriparatide, BMP-2, Corticorelin ovine triflutate, Linaclotide, Nesiritide, Lucinactant, Bivalirudin, Lepirudin, Thymalfasin, Glatiramer, Glucagon, Aviptadil, Secretin, Thymosin-b4, Teduglutide, GLP-1, GLP-2 and analoga, Plecanatide, Ecallantide, Anakinra, Disiteritide, Lixisenatide, Liraglutide, Semaglutide, Abaloparatide, Goserelin, Lanreotide, Carfilzomib, Enfuvirtide, T-20, Terlipressin, Elcatonin, Afamelanotide, Oxodotreotide, Caspofungin, Colistin, Polymyxin E, Cyclosporine, Dactinomcyin, Lyovac-Cosmegen, Degarelix, Vancomycin, Secretin, Ziconotide, Gonadorelin, Somastatin, Sincalide, Eptifibatid, Vapreotide, Triptorelin, Desmopressin, Lypressin, Atosiban, Pramintide, Pasireotide, Sandostatin, and Icatibant. The afore-mentioned peptides may be of mammalian or human origin. Also encompassed are analogues of the afore-mentioned peptides that originate from other species, for example homologues from other animals, microorganisms, virus and others.

In certain embodiments, the peptide or protein of interest is a type I secretion substrate. More than 1000 proteins are annotated or have been described as type I secretion substrates in the literature. Many of them have interesting characteristics for the biotechnological usage, in particular proteases and lipases. Suitable proteases and lipases have been described by Baumann et al. (1993) EMBO J 12, 3357-3364; and Meier et al. (2007) J. BIOL. CHEM.: 282(43), pp. 31477-31483. The content of each of these documents is incorporated by reference herein in its entirety.

In certain embodiments, the second amino acid sequence is a peptide or protein of interest which is chosen from the group consisting of MBP, lipase CalB, protease SprP, hydrolase PlaB, hydrolase PlaK, hydrolase PlbF, lipase TesA, Vif, human interferon alpha- 1, alpha-2, alpha-8, alpha- 16, alpha-21, human interferon beta, human interferon gamma, murine interferon alpha, murine interferon gamma, IFABP, Cas2, affibody protein ZA3, nisin, corticotropin release factor, amyloid- beta peptide, exenatide, Fuzeon/T20, salmon calcitonin, Mab40, Mab42, lipase LipA, SprP, the HIV- 1 protein Vif, human calcitonin, Tesamorelin, Teriparatide, BMP-2, Corticorelin ovine triflutate, Linaclotide, Nesiritide, Lucinactant, Bivalirudin, Lepirudin, Thymalfasin, Glatiramer, Glucagon, Aviptadil, Secretin, Thymosin-b4, Teduglutide, GLP-1, GLP-2 and analoga, Plecanatide, Ecallantide, Anakinra, Disiteritide, Lixisenatide, Liraglutide, Semaglutide, Abaloparatide, Goserelin, Lanreotide, Carfilzomib, Enfuvirtide, T-20, Terlipressin, Elcatonin, Afamelanotide, Oxodotreotide, Caspofungin, Colistin, Polymyxin E, Cyclosporine, Dactinomcyin, Lyovac-Cosmegen, Degarelix, Vancomycin, Secretin, Ziconotide, Gonadorelin, Somastatin, Sincalide, Eptifibatid, Vapreotide, Triptorelin, Desmopressin, Lypressin, Atosiban, Pramintide, Pasireotide, Sandostatin, and Icatibant.

The second amino acid sequence may be directly or via a linker sequence linked to the first amino acid sequence. It may be located N- or C-terminally relative to the first amino acid sequence, typically, for example but without limitation, C-terminally. This means that the N-terminus of the second amino acid sequence is linked, optionally via a linker sequence, to the C-terminus of the first amino acid sequence. However, in other embodiments, the C-terminus of the second amino acid sequence may be fused, optionally via a linker sequence, to the N-terminus of the first amino acid sequence.

In various embodiments, the linker sequence that connects the first and second amino acid sequences is also a peptide sequence and connected to the respective ends of the first and second amino acid sequence via a peptide bond. In various embodiments, the linker sequence may be 1 to 50 amino acids in length, for example 1 to 30 amino acids or 5 to 20 amino acids. As disclosed above, the first amino acid sequence together with said linker sequence may have a maximum length of 250 amino acids. In various embodiments, the linker sequence is also heterologous to the first and the second amino acid sequence and/or does not naturally occur as part of the first and/or second amino acid sequence.

The linker sequence may be functional in that it may provide for easy cleavage and separation of the first and second amino acid. To facilitate such a purpose, it can comprise or consist of a protease recognition and cleavage site. The linker may also comprise both, a linker sequence that serves only as a link and a protease cleavage site. The linker sequence may be a G-rich sequence, for example 4 or 5 consecutive G residues, optionally followed by an S residue.

The term "protease (recognition and) cleavage site" refers to a peptide sequence which can be cleaved by a selected protease thus allowing the separation of peptide or protein sequences which are interconnected by a protease cleavage site. In certain embodiments the protease cleavage site is selected from the group consisting of a Factor Xa, a tobacco edge virus (TEV) protease, a enterokinase, a SUMO Express protease, an Arg-C proteinase, an Asp-N endopeptidases, an Asp-N endopeptidase + N-terminal Glu, a caspase 1, a caspase 2, a caspase 3, a caspase 4, a caspase 5, a caspase 6, a caspase 7, a caspase 8, a caspase 9, a caspase 10, a chymotrypsin-high specificity, a chymotrypsinlow specificity, a clostripain (Clostridiopeptidase B), a glutamyl endopeptidase, a granzyme B, a pepsin, a proline-endopeptidase, a proteinase K, Welqut protease, Clean Cut protease, a staphylococcal peptidase I, a Thrombin, a Trypsin, inteins, SprB or SpIA-E from *Staphylococcus aureus,* and a Thermolysin cleavage site. In various embodiments, it may be a Factor Xa, SprB or TEV protease cleavage site. It can be preferred, in some embodiments, to design the protease recognition site such that as few amino acids as possible of the recognition and cleavage site remain attached to the peptide or protein of interest. In various embodiments, a protease recognition and cleavage site is included, the site being for example a TEV protease recognition and cleavage site. The TEV protease cleavage site typically comprises the amino acid sequence ENLYFQG/S (SEQ ID NO:64) and cleaves between the Gln (Q) and Gly/Ser (G/S) residues. In various embodiments, the P1' amino acids may be A, M or C instead of G or S.

In various embodiments, the isolated polypeptide may further comprise at least one third amino acid sequence, for example an affinity tag.

The term "affinity tag" as used herein relates to entities which are coupled to a molecule of interest and allow enrichment of the complex between the molecule of interest and the affinity tag using an affinity tag receptor. In certain embodiments affinity tags may be selected from the group consisting of the Strep-tag^{®} or Strep-tag^{®} II, the myc-tag, the FLAG-tag, the His-tag, the small ubiquitin-like modifier (SUMO) tag, the covalent yet dissociable NorpD peptide (CYD) tag, the heavy chain of protein C (HPC) tag, the calmodulin binding peptide (CBP) tag, or the HA-tag or proteins such as Streptavidin binding protein (SBP), maltose binding protein (MBP), and glutathione-S-transferase.

In various embodiments, the isolated polypeptide has relative to a polypeptide comprising a first amino acid sequence not covered by the present definition, for example a first amino acid sequence as set forth in SEQ ID NO:26, an increased renaturation efficiency. "Increased", as used in this connection, refers to yields of re-naturated polypeptides that are at least 10%, preferably at least 20% higher than those achieved with the reference sequence. Renaturation efficiency may be determined by comparing the levels of (i) renaturation of isolated polypeptides comprising as the first amino acid sequence a reference sequence and (ii) non-hydrolyzed isolated polypeptides comprising as the first amino acid sequence an amino acid sequence as described herein, after expression in form of inclusion bodies, purification and renaturation under otherwise identical conditions.

In various embodiments, the first amino acid sequence comprises as the first, N-terminal amino acid the residue M. If this is not present within the specific sequences disclosed herein, it may be artificially added, if desired, in particular to facilitate expression in a host cell.

In various embodiments, the polypeptides of the present invention consist of the elements
(1) First amino acid sequence, as defined herein;
(2) Second amino acid sequence, as defined herein;
(3) Optional linker between first and second amino acid sequence, as defined herein;
(4) Optional third amino acid sequence, as defined herein, with optional short linker sequences (1-20 amino acids) between the third amino acid sequence and any adjacent element(s).

The polypeptides of the invention may thus comprise or consist of the structure
"First amino acid sequence"-linker-"Second amino acid sequence"
   or
"Second amino acid sequence"-linker-"First amino acid sequence".

If a third amino acid sequence is present this may be attached to the N- or C-terminus of any one of these structures.

The invention further relates to the nucleic acid, in particular the isolated nucleic acid molecule, encoding the polypeptide as described above. The polypeptide comprises the first amino acid sequence and the second amino acid sequence and optionally at least one third amino acid sequence. All of these amino acid sequences are typically linked by peptide bonds and expressed as a single fusion protein. To facilitate said expression, the nucleic acid molecule comprises a first nucleotide sequence encoding the first amino acid sequence and optionally a second, third and further nucleotide sequence encoding the second, third and further amino acid sequence, with said nucleotide sequences being operably linked to allow expression of the single fusion protein comprising all afore-mentioned amino acid sequences.

The term "operably linked" in the context of nucleic acid sequences means that a first nucleic acid sequence is linked to a second nucleic acid sequence such that the first nucleic acid sequence is placed in a functional relationship with the second nucleic acid sequence. For instance, a promoter sequence is operably linked to a coding sequence of a heterologous gene if the promoter can initiate the transcription of the coding sequence. In a further context, a sequence encoding for the first amino acid sequence is linked such to a second amino acid sequence encoding for a peptide or protein of interest, that if the two sequences are translated a single peptide/protein chain is obtained.

In certain embodiments, the above defined nucleic acid molecules may be comprised in a vector, for example a cloning or expression vector. Generally, the nucleic acid molecules of the invention can also be part of a vector or any other kind of cloning vehicle, including, but not limited to a plasmid, a phagemid, a phage, a baculovirus, a cosmid, or an artificial chromosome. Generally, a nucleic acid molecule disclosed in this application may be "operably linked" to a regulatory sequence (or regulatory sequences) to allow expression of this nucleic acid molecule.

Such cloning vehicles can include, besides the regulatory sequences described above and a nucleic acid sequence of the present invention, replication and control sequences derived from a species compatible with the host cell that is used for expression as well as selection markers conferring a selectable phenotype on transformed or transfected cells. Large numbers of suitable cloning vectors are known in the art, and are commercially available.

In certain embodiments the nucleic acid molecules disclosed herein are comprised in a cloning vector. In some embodiments the nucleic acid molecules disclosed herein are comprised in an expression vector. The vectors may comprise regulatory elements for replication and selection markers. In certain embodiments, the selection marker may be selected from the group consisting of genes conferring ampicillin, kanamycin, chloramphenicol, tetracycline, blasticidin, spectinomycin, gentamicin, hygromycin, and zeocin resistance. In various other embodiments, the selection may be carried out using antibiotic-free systems, for example by using toxin/antitoxin systems, cer sequence, triclosan, auxotrophies or the like. Suitable methods are known to those skilled in the art.

The above-described nucleic acid molecule of the present invention, comprising a nucleic acid sequence encoding for a protein of interest, if integrated in a vector, must be integrated such that the peptide or protein of interest can be expressed. Therefore, a vector of the present invention comprises sequence elements which contain information regarding to transcriptional and/or translational regulation, and such sequences are "operably linked" to the nucleotide sequence encoding the polypeptide. An operable linkage in this context is a linkage in which the regulatory sequence elements and the sequence to be expressed are connected in a way that enables gene expression. The precise nature of the regulatory regions necessary for gene expression may vary among species, but in general these regions comprise a promoter which, in prokaryotes, contains both the promoter per se, i.e. DNA elements directing the initiation of transcription, as well as DNA elements which, when transcribed into RNA, will signal the initiation of translation. Such promoter regions normally include 5' non-coding sequences involved in initiation of transcription and translation, such as the -35/- 10 boxes and the Shine-Dalgarno element in prokaryotes or the TATA box, CAAT sequences, and 5'- capping elements in eukaryotes. These regions can also include enhancer or repressor elements as well as translated signal and leader sequences for targeting the native polypeptide to a specific compartment of a host cell.

In addition, the 3' non-coding sequences may contain regulatory elements involved in transcriptional termination, polyadenylation or the like. If, however, these termination sequences are not satisfactory functional in a particular host cell, then they may be substituted with signals functional in that cell.

In various embodiments, a vector comprising a nucleic acid molecule of the invention can therefore comprise a regulatory sequence, preferably a promoter sequence. In certain embodiments, the promoter is identical or homologous to promoter sequences of the host genome. In such cases endogenous polymerases may be capable to transcribe the nucleic acid molecule sequence comprised in the vector. In various embodiments, the promoter is selected from the group of weak, intermediate and strong promoters, preferably from weak to intermediate promoters.

In another preferred embodiment, a vector comprising a nucleic acid molecule of the present invention comprises a promoter sequence and a transcriptional termination sequence. Suitable promoters for prokaryotic expression are, for example, the araBAD promoter, the tet-promoter, the lacUV5 promoter, the CMV promo tor, the EF1 alpha promotor, the AOX1 promotor, the tac promotor, the T7promoter, or the lac promotor. Examples of promoters useful for expression in eukaryotic cells are the SV40 promoter or the CMV promoter. Furthermore, a nucleic acid molecule of the invention can comprise transcriptional regulatory elements, e.g., repressor elements, which allow regulated transcription and translation of coding sequences comprised in the nucleic acid molecule. Repressor element may be selected from the group consisting of the Lac-, AraC-, or MaIR-repressor.

The vector may be effective for prokaryotic or eukaryotic protein expression. In particular, the nucleic acid molecules of the present invention may be comprised in a vector for prokaryotic protein expression. Such vector sequences are constructed such that a sequence of interest can easily be inserted using techniques well known to those skilled in the art. In certain embodiments, the vector is selected from the group consisting of a pET-vector, a pBAD-vector, a pK184-vector, a pMONO-vector, a pSELECT-vector, pSELECT-Tag-vector, a pVITRO-vector, a pVIVO-vector, a pORF-vector, a pBLAST-vector, a pUO-vector, a pDUO-vector, a pZERO-vector, a pDeNy-vector, a pDRIVE-vector, a pDRIVE-SEAP-vector, a HaloTag^{®}Fusion- vector, a pTARGET^{™}-vector, a Flexi^{®}-vector, a pDEST-vector, a pHIL-vector, a pPIC-vector, a pMET-vector, a pPink-vector, a pLP -vector, a pTOPO-vector, a pBud-vector, a pCEP-vector, a pCMV-vector, a pDisplay-vector, a pEF-vector, a pFL-vector, a pFRT-vector, a pFastBac-vector, a pGAPZ-vector, a plZ/V5-vector, a p3S-vector, a plAR-vector, pSEC, pMS, a pSU2726-vector, a pLenti6 -vector, a pMIB-vector, a pOG-vector, a pOpti-vector, a pREP4-vector, a pRSET-vector, a p SCREEN- vector, a pSecTag-vector, a pTEFI -vector, a pTracer-vector, a pTrc-vector, a pUB6-vector, a pVAXI-vector, a pYC2-vector, a pYES2-vector, a pZeo-vector, a pcDNA-vector, a pFLAG-vector, a pTAC-vector, a pT7-vector, a gateway^{®}-vector, a pQE-vector, a pLEXY-vector, a pRNA-vector, a pPK-vector, a pUMVC-vector, a pLIVE-vector, a pCRUZ-vector, a Duet-vector, and other vectors or derivatives thereof.

The vectors of the present invention may be chosen from the group consisting of high, medium and low copy vectors.

The above described vectors of the present invention may be used for the transformation or transfection of a host cell in order to achieve expression of a peptide or protein which is encoded by an above described nucleic acid molecule and comprised in the vector DNA. Thus, in a further aspect, the present invention also relates to a host cell comprising a vector or nucleic acid molecule as disclosed herein.

Also contemplated herein are host cells, which comprise a nucleic acid molecule as described herein integrated into their genomes. The skilled person is aware of suitable methods for achieving the nucleic acid molecule integration. For example, the molecule may be delivered into the host cells by means of liposome transfer or viral infection and afterwards the nucleic acid molecule may be integrated into the host genome by means of homologous recombination. In certain embodiments, the nucleic acid molecule is integrated at a site in the host genome, which mediates transcription of the peptide or protein of the invention encoded by the nucleic acid molecule. In various embodiments, the nucleic acid molecule further comprises elements which mediate transcription of the nucleic acid molecule once the molecule is integrated into the host genome and/or which serve as selection markers.

In certain embodiments, the nucleic acid molecule of the present invention is transcribed by a polymerase natively encoded in the host genome. In various embodiments, the nucleic acid molecule is transcribed by a RNA-polymerase which is non-native to the host genome. In such embodiments, the nucleic acid molecule of the present invention may further comprise a sequence encoding for a polymerase and/or the host genome may be engineered or the host cell may be infected to comprise a nucleic acid sequence encoding for an exogenous polymerase. The host cell may be specifically chosen as a host cell capable of expressing the gene. In addition or otherwise, in order to produce the isolated polypeptide of the invention, the nucleic acid coding for it can be genetically engineered for expression in a suitable system. Transformation can be performed using standard techniques (Sambrook, J. et al. (2001), Molecular Cloning: A Laboratory Manual, 3rd Ed., Cold Spring Harbor Laboratory Press, Cold Spring Harbor, NY).

Prokaryotic or eukaryotic host organisms comprising such a vector for recombinant expression of the polypeptide as described herein form also part of the present invention. Suitable host cells can be prokaryotic cells. In certain embodiments the host cells are selected from the group consisting of gram positive and gram negative bacteria. In some embodiments, the host cell is a gram negative bacterium, such as E.coli. In certain embodiments, the host cell is E. coli, in particular E. coli BL21 (DE3) or other E. coli K12 or E. coli B834 derivatives. In further embodiments, the host cell is selected from the group consisting of Escherichia coli (E. coli), Pseudomonas, Serratia marcescens, Salmonella, Shigella (and other enterobacteriaceae), Neisseria, Hemophilus, Klebsiella, Proteus, Enter obacter, Helicobacter, Acinetobacter, Moraxella, Helicobacter, Stenotrophomonas, Bdellovibrio, Legionella, acetic acid bacteria, Bacillus, Bacilli, Carynebacterium, Clostridium, Listeria, Streptococcus, Staphylococcus, and Archaea cells. Suitable eukaryotic host cells are among others CHO cells, insect cells, fungi, yeast cells, e.g., Saccharomyces cerevisiae, S. pombe, Pichia pastoris.

In certain embodiments, the host cell is a prokaryotic cell, such as *E.coli,* in particular *E.coli* BL21 (DE3), E. coli BL21, E. coli K12, E. coli BLR, E. coli BL21 Al, E. coli BL21 pLysS, E. coli XL1 and E. coli DH5a. Further suitable *E.coli* strains include, but are not limited to DH1, DH5a, DM1, HB101, JmIOI-110, Rosetta(DE3)pLysS, SURE, TOP10, XLI-Blue, XL2-Blue and XLIO-Blue strains.

The transformed host cells are cultured under conditions suitable for expression of the nucleotide sequence encoding the polypeptide of the invention. In certain embodiments, the cells are cultured under conditions suitable for expression of the nucleotide sequence encoding a polypeptide of the invention.

For producing the recombinant peptide or protein of interest in form of the fusion proteins described herein, a vector of the invention can be introduced into a suitable prokaryotic or eukaryotic host organism by means of recombinant DNA technology (as already outlined above). For this purpose, the host cell is first transformed with a vector comprising a nucleic acid molecule according to the present invention using established standard methods (Sambrook, J. et al. (2001), supra). The host cell is then cultured under conditions, which allow expression of the heterologous DNA and thus the synthesis of the corresponding polypeptide. Subsequently, the polypeptide is recovered ("isolated") either from the cell or from the cultivation medium.

For expression of the peptides and proteins of the present invention several suitable protocols are known to the skilled person. The expression of a recombinant polypeptide of the present invention may be achieved by the following method comprising: (a) introducing a nucleic acid molecule or vector of the invention into a host cell, wherein the nucleic acid molecule or vector encodes the recombinant polypeptide; and (b) cultivating the host cell in a culture medium under conditions that allow expression of the recombinant polypeptide.

Step (a) may be carried out by using suitable transformation and transfection techniques known to those skilled in the art. These techniques are usually selected based on the type of host cell into which the nucleic acid is to be introduced. In some embodiments, the transformation may be achieved using electroporation or heat shock treatment of the host cell.

Step (b) may include a cultivation step that allows growth of the host cells. Alternatively, such step allowing growth of the host cells and a step that allows expression of the polypeptide may be performed separately in that the cells are first cultivated such that they grow to a desired density and then they are cultivated under conditions that allow expression of the polypeptide. The expression step can however still allow growth of the cells.

The method may further include a step of recovering the expressed polypeptide. The polypeptide may be recovered from the growth medium, if it is secreted, or from the cells or both. Preferably the polypeptide is recovered from the cells. The recovery of the polypeptide may include various purification steps.

Generally, any known culture medium suitable for growth of the selected host may be employed in this method. In various embodiments, the medium is a rich medium or a minimal medium. Also contemplated herein is a method, wherein the steps of growing the cells and expressing the peptide or protein comprise the use of different media. For example, the growth step may be performed using a rich medium which is replaced by a minimal medium in the expression step. In certain cases, the medium is selected from the group consisting of LB medium, TB medium, 2YT medium, synthetical medium and minimal medium.

In various preferred embodiments of the invention, the polypeptide of the invention is not secreted. In such embodiments, it may preferably be expressed in form of inclusion bodies (IBs). In many cases, it may be useful to express the polypeptide of the invention in such an insoluble form, for example in cases where the peptide of interest is rather short, normally soluble and/or subject to proteolytic degradation within the host cell. Production of the peptide in insoluble form both facilitates simple recovery and additionally protects the peptide from the undesirable proteolytic degradation. In such embodiments, the first amino acid sequence may serve as a solubility tag, i.e., an inclusion body (IB) tag, that induces IB formation. Calcium ion (or earth alkaline metal ion) binding to the GG repeat(s) or any other change of environment may later catalyze the GG-repeat-facilitated folding of the fusion polypeptide into the native, active conformation, optionally with the calcium/earth alkaline metal ions acting as a folding helper/chaperone. Renaturation/refolding can, in certain instances, surprisingly also occur in the absence of calcium and/or earth alkaline metal ions, but is still critically dependent on the presence of the GG repeat elements disclosed herein.

It has been described above, that the inventor has found a variety of first amino acid sequences that increase renaturation efficiency in such methods compared to the respective full-length proteins they are derived from or natural fragments thereof. It has further surprisingly found that even comparably short first amino acid sequences can successfully induce proper renaturation of a comparably long second amino acid sequence, with said second amino acid sequence not comprising any such refolding sequence motifs, such as GG repeats.

The terms "inclusion body" or "IB", as interchangeably used herein, relate to nuclear or cytoplasmic aggregates of substances, for instance proteins. IBs are undissolved and have a non-unit lipid membrane. In the method of the present invention, the IBs mainly consist of the fusion protein comprising at least one peptide/protein of interest and the first amino acid sequence, as defined herein.

In various embodiments, in particular where expression of the polypeptide of the invention in form of IBs is desired, the expression of the endogenous ABC transporter gene, the endogenous MFP gene and/or the endogenous OMP gene of the T1 SS or the activity of the corresponding gene products in the host cell is inhibited. In various embodiments, the host cell does not express endogenous ABC transporter, endogenous MFP and/or endogenous OMP of the T1 SS. The host cell may be engineered accordingly.

Methods to inhibit the expression of genes such as their deletion or insertion of nucleotide sequences destroying the integrity of the promoter sequence or the gene itself are known in the art. A preferred gene expression activity after deletion or disruption may be less than 35 %, 30 %, 25 %, 20 %, 15 %, 10 % or 5 % of the activity measured in untreated cells. In other various embodiments of the invention, the endogenous ABC transporter, the endogenous MFP and/or the endogenous OMP of the type 1 secretion system are inhibited by antibodies or small molecule inhibitors. In preferred embodiments of the invention, the ABC transporter activity is inhibited by orthovanadate or an ATP homologous inhibitor such as 8-azido-ATP. Such ATP mimetics are known in the art. The preferred protein activity after inhibitor treatment may be less than 35 %, 30 %, 25 %, 20 %, 15 %, 10 % or 5 % of the activity measured in untreated cells. In other embodiments of the invention, the transport is inhibited or blocked by the polypeptide of the invention itself, for example by over-expressing it.

In various embodiments of the afore-mentioned methods, the polypeptide of the invention is recovered from the host cells in form of insoluble inclusion bodies, for example by any of the purification methods disclosed herein.

Renaturation may then be effected by exposing the isolated/purified polypeptide to renaturation conditions. In some instances, this may include buffer conditions wherein the so-called refolding buffer comprises at least 0.01, more preferably 0.01-40 mM of alkaline earth metal ions, in particular Ca²⁺. By virtue of the presence of GG repeats in the first amino acid sequence, such treatment triggers refolding of the polypeptide into its functional (native) conformation. Interestingly, the refolding of the first amino acid sequence also induces proper folding of the peptide/polypeptide of interest fused thereto. It has surprisingly been found by the inventors that such refolding can, in various embodiments, also occur in the absence of calcium or other earth alkaline metal ions. These are therefore not mandatory components of the refolding buffer. This may be particularly advantageous for peptides and proteins that are susceptible to adverse effects by the presence of calcium or other earth alkaline metal ions. In various embodiments, the renaturation/refolding thus occurs in the absence of calcium and/or earth alkaline metal ions in general. It may be preferred that the refolding occurs under reducing conditions, for example by using a reduction agent, such as TCEP ((tris(2-carboxyethyl)phosphine)).

The general advantages of expression of a fusion protein in form of inclusion bodies are set forth in greater detail in WO 2014/170430 A1, which is herewith included by reference in its entirety.

In various embodiments, the method also encompasses the purification the recombinant polypeptide, wherein the recombinant polypeptide is purified using a method selected from affinity chromatography, ion exchange chromatography, reverse phase chromatography, size exclusion chromatography, and combinations thereof.

In several embodiments, the method may comprise the treatment of the recombinant polypeptide with a protease suitable for cleavage of a protease cleavage site within the recombinant polypeptide. In preferred embodiments, the recombinant polypeptide is purified and, optionally, renaturated prior to proteolytic cleavage using one or more methods disclosed above. Also after cleavage of the recombinant peptide or protein, the method may comprise a further purification step as defined above. Thus, in some embodiments the recombinant polypeptide is purified, subjected to proteolytic cleavage and the peptide or protein of interest is further purified. In other embodiments, the protease may be co-expressed or added to the cultivation medium or expressed by co-cultivated microorganisms, such that cleavage occurs before purification. However, since renaturation of the peptide or polypeptide of interest is mediated by the first amino acid sequence, such strategy may not be preferred.

In a further aspect, the present invention relates to the use of a vector or nucleic acid molecule as disclosed herein for the expression of a recombinant polypeptide.

A method for expression of a recombinant peptide or protein using the above-described nucleic acid molecules may comprise the steps of:
(a) introducing a nucleic acid molecule or a vector as described above into a suitable host cell, wherein the nucleic acid molecule or vector encodes the recombinant polypeptide; and
(b) cultivating the host cell in a culture medium under conditions that allow expression of the recombinant polypeptide.

The expression of the recombinant polypeptide in step (b) may be in form of inclusion bodies.

The method can further be defined as the other methods of the invention described above. Specifically, the method may further comprise recovering the expressed peptide or protein from the host cell and/or the culture medium. In addition, the host cell may be a prokaryotic cell; and/or the expression may be performed in minimal culture medium; and/or the recombinant polypeptide may be purified using a method selected from affinity chromatography, ion exchange chromatography, reverse phase chromatography, size exclusion chromatography, and combinations thereof; and/or the method may comprises treatment of the recombinant polypeptide with a protease suitable for cleavage of a protease cleavage site within the recombinant polypeptide; and/or the method may comprise a cleavage step followed by purification of the recombinant polypeptide.

In various embodiments, the polypeptide is recovered in form of inclusion bodies and, after purification, exposed to a refolding buffer. The buffer may comprise at least 0.01, more preferably 0.01-40 mM of earth alkaline metal ions, such as Ca²⁺. In other instances, the refolding buffer is free of calcium and other earth alkaline metal ions. As already detailed above, it has surprisingly been found that the first amino acid sequences disclosed herein also provide for good refolding under conditions without any calcium or earth alkaline metal ions. This is counterintuitive, since it was previously assumed that GG repeats only act by virtue of their interaction with calcium ions. In various embodiments, the methods of the invention thus may also comprise a step of re-solubilizing the peptide/protein and/or reconstituting/refolding it under suitable conditions that may be conditions with or without calcium. Said step is also referred to herein as "renaturing" step. "Without calcium", as used in this context, means that the concentration of calcium is so low that it has virtually no impact on the folding. Such concentration may be less than 0.01 mM, less than 0.001 mM or les than 0.0001 mM.

In still another aspect, the present invention is therefore also directed to methods for renaturing the isolated polypeptide of the invention, wherein the method may comprise contacting, for example in form of re-solubilizing, the isolated polypeptide with a suitable medium, typically an aqueous medium, optionally comprising earth alkaline metal ions, for example the refolding buffer disclosed above. Such a medium allows the GG repeats (comprised in the first amino acid sequence) to induce conformational changes that in turn then facilitate refolding/renaturation of the fused heterologous peptide or polypeptide (second amino acid sequence).

In still another aspect, the present invention also relates to the use of an isolated polypeptide of the invention for facilitating the production of a recombinant peptide or protein. Said recombinant peptide of protein is part of the isolated polypeptide (in form of the second amino acid sequence), which is expressed as a fusion protein. In such embodiments, the first amino acid sequences disclosed herein may be used to facilitate renaturation/refolding of said recombinant peptide or polypeptide. Said recombinant peptide or protein is identical with the peptide or polypeptide of interest as referred to herein and may thus consist of the second amino acid sequence disclosed herein. As stated above, said use is preferably directed to recombinant polypeptides expressed as fusion proteins in a suitable expression system.

### EXAMPLES

### Materials and methods

### Expression host: Escherichia coli BL21 (DE3) (Novagen)

All oligonucleotides were purchased from Microsynth Seqlab GmbH.

Codon optimized nucleotide sequences encoding peptides were purchased from Thermo Fisher Scientific. All enzymes were purchased from NEB, Clontech, Invitrogen or Fermentas.

### Expression protocol

1. Transformation of chemically competent cells with an expression vector encoding for a first amino acid sequence that is a scaffold derived from HlyA1 with the amino acid sequence set forth in SEQ ID NO:50691 with the GG repeat elements, as disclosed below, being inserted between positions 10 and 11 of SEQ ID NO:50691 and the peptide/polypeptide of interest (second amino acid sequence; as a fusion protein) and plating of the transformed cells on LB agar plates comprising suitable antibiotic(s) for selection of the transformed cells.
2. Incubation of the agar plates over night at 37°C.
3. Inoculation of 2YT medium comprising antibiotics with a single colony from the agar plate for an overnight culture.
4. Incubation at 37°C and shaking of the culture over night.
5. Inoculation of the main culture comprising 2x YT medium (16 g tryptone/peptone from casein (Roth, #8952.2), 10 g yeast extract (Roth, #2363.2), 5 g NaCl (Roth, #3957.1), ad 1 L demineralized water with the overnight culture resulting in an OD600 of 0.01 - 0.2 (flasks with baffles)
6. Incubation of the culture at 37°C at different rpm
7. Induction of the expression the peptide or protein of interest with 1 mM IPTG at an OD600 of 0.4-1.0.
8. Incubation of the cultures for 3 hrs.
9. Culture samples were taken at 0 hrs and 3 hrs post induction and centrifuged for 10 min., 13,000 x g, RT.
10. Cell samples were resuspended in water to adjust an OD of 5.0, mixed 4:1 with 5x SDS loading dye and heated (95°C, 10 min).
11. 20 µL samples were loaded on 15% SDS-PAGE gels and SDS-PAGE analysis was performed at 160 V for about 45 min.

### Example 1: Optimized GG repeat sequences for renaturation

Fusion constructs of the modified HlyA1 scaffold (SEQ ID NO:50691) with different GG repeat sequences being inserted between positions 10 and 11 (cf. Table 1) and an N-terminal M as first amino acid sequence with a polypeptide of interest (hEGF; SEQ ID NO:50696) C-terminally fused thereto via a linker comprising a 5 amino acid long G rich sequence (SEQ ID NO:50693) and a TEV protease site (SEQ ID NO:50694) were designed. Fusion constructs were expressed as inclusion bodies (lbs) in *E.coli* BL21 (DE3), lbs were prepared via BugBuster Kit and the lbs dissolved in 6 M GuHCl (1:4 w/v). After solubilization overnight, 1 mM stock solution of each construct was set in 6 M GuHCl and 5 mM TCEP (Tris(2-carboxyethyl)phosphine) added. Renaturation was performed at room temperature for 20 min setting a final concentration of 0.05 mM in a total volume of 500 µL renaturation buffer with calcium ions (10 mM Tris-HCI, 120 mM NaCl, pH 7.3 supplemented with 20 mM CaCl₂) or without calcium ions (HEPES + EDTA). The renaturation efficiency was determined in the cleared supernatant by UV/Vis spectroscopy.

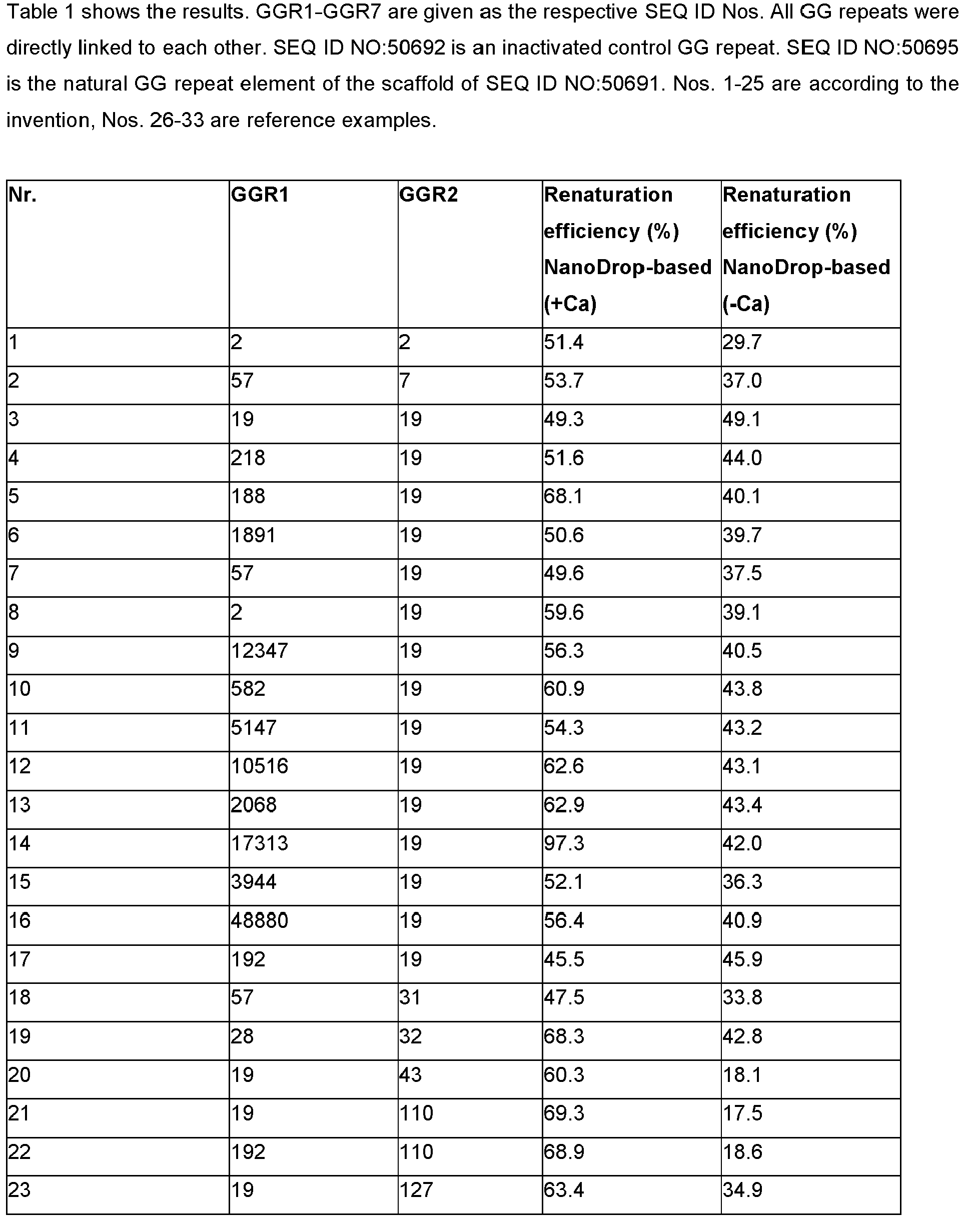

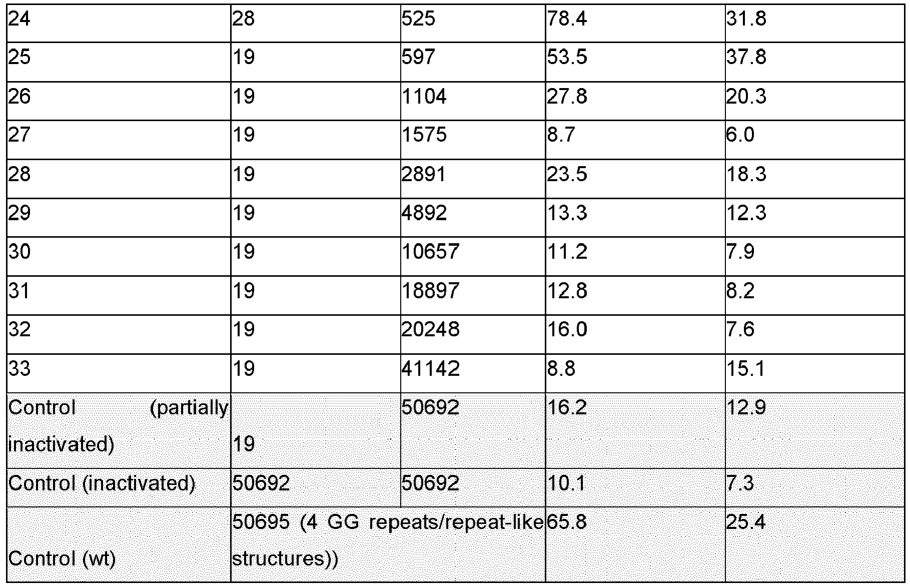

It can be seen from the data in Table 1 that the inventive fusion constructs provided for similar or better renaturation efficiency compared to a longer control construct (wt; or controls with 1 or 2 inactivated GG repeats). It needs to be noted that for first amino acid sequence (with two GG repeat sequences) even renaturation efficiencies slightly lower than the wt control (with four GG repeats/repeat-like sequences) may still be advantageous, as the proportion of the peptide/polypeptide of interest in the construct is significantly higher.

All documents cited herein, are hereby incorporated by reference in their entirety. The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

## Claims

1. Isolated polypeptide comprising a first and a second amino acid sequence, wherein
(a) the first amino acid sequence
(a1) comprises in N- to C-terminus orientation the general amino acid sequence element
GGR1-Linker-GGR2
wherein GGR1 is a GG repeat sequence of the consensus sequence GGxGxDxUx, wherein each x can independently be any amino acid and U is independently a hydrophobic, large amino acid selected from F, V, A, Y, I, L and M;
wherein GGR2 is a GG repeat sequence selected from any one of the amino acid sequences set forth in SEQ ID Nos. 1-1000, preferably SEQ ID Nos. 1-500;
wherein "Linker" is independently either a peptide bond or an amino acid sequence of 1 to 25 amino acids, preferably a peptide bond;
(a2) wherein the first amino acid sequence is 18 to 300, preferably 30 to 200 amino acids in length; and
(a3) wherein the first amino acid sequence and/or the amino acid sequence element GGR1-Linker-GGR2 is a non-natural sequence element;
(b) the second amino acid sequence is at least one peptide or polypeptide of interest, and
(c) wherein the first and second amino acid sequence are heterologous to each other.

2. The isolated polypeptide of claim 1, wherein GGR2 is selected from any one of the amino acid sequences set forth in SEQ ID Nos. 2, 7, 19, 43, 110, 127, 525, and 597.

3. The isolated polypeptide of claim 1 or 2, wherein the first amino acid sequence comprises at least 3, at least 4, at least 5, at least 6, at least 7 or at least 8 GG repeat sequences of the general consensus sequence GGxGxDxUx, wherein any third and further GG repeat sequence(s) are located C-terminal to the sequence element GGR1-Linker-GGR2 such that the first amino acid sequence optionally comprises the general structure, from N- to C-terminus,
(vii) GGR1-Linker-GGR2-Linker-GGR3; or
(viii) GGR1-Linker-GGR2-Linker-GGR3-Linker-GGR4; or
(ix) GGR1-Linker-GGR2-Linker-GGR3-Linker-GGR4-Linker-GGR5; or
(x) GGR1-Linker-GGR2-Linker-GGR3-Linker-GGR4-Linker-GGR5-Linker-GGR6; or
(xi) GGR1-Linker-GGR2-Linker-GGR3-Linker-GGR4-Linker-GGR5-Linker-GGR6-Linker-GGR7; or
(xii) GGR1-Linker-GGR2-Linker-GGR3-Linker-GGR4-Linker-GGR5-Linker-GGR6-Linker-GGR7-Linker-GGR8
wherein GGR3, GGR4, GGR5, GGR6, GGR7 and GGR8 are each independently GG repeats of the consensus sequence GGxGxDxUx, wherein each x can independently be any amino acid and U is independently a hydrophobic, large amino acid selected from F, V, A, Y, I, L and M, and wherein each "Linker" is independently either a peptide bond or an amino acid sequence of 1 to 25 amino acids.

4. The isolated polypeptide of any one of claims 1 to 3, wherein the general sequence element GGR1-Linker-GGR2 or any of the general structures (i)-(vi) according to claim 3 is flanked by additional N- and/or C-terminal amino acid sequences, optionally each being up to 150 amino acids in length.

5. The isolated polypeptide of claim 4, wherein
(1a) the N-terminal flanking sequence is 1 to 100 amino acids in length, preferably 1 to 80, 1 to 70, 5 to 60, 5 to 50, 5 to 40, 5 to 30, 8 to 20, 10 to 15 or 10 amino acids in length; and/or
(1b) the N-terminal flanking sequence comprises or consist of the amino acid sequence set forth in SEQ ID NO:50688; and/or
(2a) the C-terminal flanking sequence is 10 to 150 amino acids in length, preferably 20 to 120, 30 to 120, 40 to 120, 50 to 120, 60 to 120, 70 to 120, 80 to 120, 90 to 120, 100 to 120 or 110 to 120 amino acids in length; and/or
(2b) the C-terminal flanking sequence comprises or consist of the amino acid sequence set forth in SEQ ID NO:50689; and/or
(3) the N- and/or C-terminal flanking sequences are scaffold sequences derived from a natural occurring RTX protein, in which any one or more of the naturally-occurring GG repeat sequences have been replaced by the general sequence element GGR1-Linker-GGR2 or any of the general structures (i)-(vi) according to claim 3; and/or
(4) the scaffold sequence is derived from hemolysin A protein, preferably hemolysin A1 having the amino acid sequence set forth in SEQ ID NO:50690 or a variant or fragment thereof, more preferably the amino acid sequence as set forth in SEQ ID NO:50691 or a variant or fragment thereof, wherein the general sequence element GGR1-Linker-GGR2 or any of the general structures (i)-(vi) according to claim 3 are inserted between positions 10 and 11 using the positional numbering of SEQ ID NO:50691.

6. The isolated polypeptide of any one of claims 1 to 5, wherein
(1) U in the consensus sequence GGxGxDxUx is selected from F, V, I, L, M and Y or F, I, L, M and Y; and/or
(2) the consensus sequence of the GG repeat sequences is GGX¹GX²DX³UX⁴, wherein X¹, X², X³ and X⁴ may be any amino acid with the exception of P, preferably X¹ is selected from G, A, L, V, I, M, F, S, T, Q; Y, K, R, D, E, N, and Y, preferably from G, A, E, S, T, Q, L, R and D, more preferably from G, A, E, S and T; and/or X² is selected from N, D, A, G, S, H, T, E, M, R and H, preferably from N, D, A, S, and H, more preferably from N, D, A, and S; and/or X³ is selected from A, K, V, L, I, F, M, R, Y, S, L, T, V, Q, N, D, E, and H, preferably from T, R, V, L, S, I, A, Y, Q, D and H, more preferably from T, R, V, L, S and I, even more preferably from T, R and V; and/or X⁴ is selected from W, K, R, Y, V, L, T, H, D, A, M, E, F, I, S, N and Q, preferably from V, L, I, F, S, R, N, Y and T; and/or
(3) GGR1 and optionally any further GG repeat sequence are selected from the amino acid sequences set forth in SEQ ID Nos. 1-50687.

7. The isolated polypeptide of any one of claims 1 to 6, wherein
(a) GGR2 is the amino acid set forth in SEQ ID NO:19 and GGR1 has the amino acid sequence set forth in any one of SEQ ID Nos. 2, 19, 218, 188, 1891, 57, 12347, 582, 8665, 5147, 10516, 2068, 3426, 17313, 3944, 48880, 192;
(b) GGR2 is the amino acid sequence set forth in SEQ ID NO:2 and GGR1 has the amino acid sequence set forth in SEQ ID NO:2;
(c) GGR2 is the amino acid sequence set forth in SEQ ID NO:7 and GGR1 has the amino acid sequence set forth in SEQ ID NO:57;
(d) GGR2 is the amino acid sequence set forth in SEQ ID NO:31 and GGR1 has the amino acid sequence set forth in SEQ ID NO:57;
(e) GGR2 is the amino acid sequence set forth in SEQ ID NO:32 and GGR1 has the amino acid sequence set forth in SEQ ID NO:28;
(f) GGR2 is the amino acid sequence set forth in SEQ ID NO:43 and GGR1 has the amino acid sequence set forth in SEQ ID NO:19;
(g) GGR2 is the amino acid sequence set forth in SEQ ID NO:110 and GGR1 has the amino acid sequence set forth in SEQ ID NO:19 or 192;
(h) GGR2 is the amino acid sequence set forth in SEQ ID NO:127 or 597 and GGR1 has the amino acid sequence set forth in SEQ ID NO:19; or
(i) GGR2 is the amino acid sequence set forth in SEQ ID NO:525 and GGR1 has the amino acid sequence set forth in SEQ ID NO:28.

8. The isolated polypeptide of claim 7, wherein "Linker" between GGR1 and GGR2 is a peptide bond.

9. The isolated polypeptide of any one of the preceding claims, wherein
(1) the isolated polypeptide is a fusion protein; and/or
(2) the second amino acid sequence is 10 to 1000 amino acids in length, preferably 15 to 500 amino acids in length; and/or
(3) the second amino acid sequence is N-terminal or C-terminal to the first amino acid sequence, preferably C-terminal, wherein the second amino acid sequence is optionally linked directly or via a linker sequence to the N- or C-terminal end of the first amino acid sequence, the linker sequence optionally being 1 to 30 amino acids in length and preferably comprising a protease recognition and cleavage site, the protease recognition and cleavage site preferably being a TEV protease recognition and cleavage site; and/or
(4) the isolated polypeptide has a total length of up to 700 amino acids, preferably up to 500 amino acids, more preferably up to 350 amino acids.

10. Nucleic acid encoding the polypeptide of any one of claims 1 to 9.

11. Vector comprising a nucleic acid molecule according to claim 10, wherein the vector preferably is a plasmid.

12. Host cell comprising a nucleic acid molecule according to claim 10 or a vector according to claim 11, the host cell preferably being a prokaryotic host cell.

13. Method for the production of a polypeptide of any one of claims 1 to 9, comprising
(1) cultivating the host cell of claim 12 under conditions that allow the expression of the polypeptide;
(2) isolating the expressed polypeptide from the host cell, wherein the expression of the polypeptide in the host cell optionally is in insoluble form, preferably in form of inclusion bodies; and
(3) renaturing the isolated polypeptide.

14. Method for renaturing the isolated polypeptide of any one of claims 1 to 9 or obtained according to the method of claim 13, wherein the method comprises dissolving or dispersing the isolated polypeptide in an aqueous medium suitable for renaturation, optionally an aqueous medium comprising earth alkaline metal ions, preferably calcium ions.

15. Use of the isolated polypeptide of any one of claims 1-9 for expression and renaturation of the second amino acid sequence in form of a fusion protein, wherein the expression is in a recombinant host cell in form of inclusion bodies.
